(19) Europäisches Patentamt
European Patent Office
Office européen des brevets

(11) **EP 4 223 147 B1**

(12) **EUROPEAN PATENT SPECIFICATION**

(45) Date of publication and mention
of the grant of the patent:
**30.10.2024 Bulletin 2024/44**

(21) Application number: **22155569.1**

(22) Date of filing: **08.02.2022**

(51) International Patent Classification (IPC):
*A23N 17/00* (2006.01)    *B01F 35/213* (2022.01)
*B01F 35/21* (2022.01)

(52) Cooperative Patent Classification (CPC):
**A23N 17/007; G01N 22/00; G01N 33/02**

(54) **PROCESS AND APPARATUS FOR MIXING A FOOD PRODUCT FOR FARM ANIMALS**

VERFAHREN UND VORRICHTUNG ZUM MISCHEN EINES NAHRUNGSMITTELPRODUKTS FÜR NUTZTIERE

PROCÉDÉ ET APPAREIL PERMETTANT DE MÉLANGER UN PRODUIT ALIMENTAIRE POUR ANIMAUX D'ÉLEVAGE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**09.08.2023 Bulletin 2023/32**

(73) Proprietor: **Faresin Industries S.p.A.**
**36042 Breganze VI (IT)**

(72) Inventors:
• **FARESIN, Sante**
  **36042 Breganze (VI) (IT)**
• **FARESIN, Silvia**
  **36042 Breganze (VI) (IT)**
• **FARESIN, Giulia**
  **36042 Breganze (VI) (IT)**
• **ANDRIGHETTO, Igino**
  **36100 Vicenza (VI) (IT)**
• **SERVA, Lorenzo**
  **35030 Rubano (PD) (IT)**
• **MAGRIN, Luisa**
  **35010 Villa del Conte (PD) (IT)**

(74) Representative: **Gallo, Luca et al**
**Gallo & Partners S.r.l.**
**Via Rezzonico, 6**
**35131 Padova (IT)**

(56) References cited:
WO-A1-2008/055456    WO-A1-2017/051424
WO-A2-2008/063661    DE-A1- 102010 033 886
US-B1- 8 967 851

# EP 4 223 147 B1

## Description

<u>Field of application</u>

The present invention refers to a process and to an apparatus for mixing a food product for farm animals. The present process and apparatus are inserted in the field of zootechnics and are advantageously intended to be employed, e.g. by diet specialists, formula specialists or stall veterinarians, in intensive farming of animals such as cattle, pigs, goats, sheep, buffalo, etcetera.

In particular, the process and the apparatus, object of the present invention, are adapted to automatically mix a mixture of nutrient elements which constitute the food product distributed to animals up to attaining uniformity.

<u>State of the art</u>

Intensive (or industrial) animal farming is generally placed in stalls normally provided with one or more corridors, along the sides of which the animal food troughs are placed.

In the stalls of intensive (or industrial) animal farming, the animals are generally fed with a food product (termed in the jargon of the field unifeed or TMR (Total Mixed Ratio)) constituted by a mixture of nutrient elements, such as hay, forage (like corn, sorghum, etc.) and concentrated foods (come soy grains, corn grains, etc.).

Feeding the animals by means of the aforesaid mixture allows the simultaneous administration of all the nutrient elements, which allows maximizing the productive efficiency, reducing to the minimum the risk of onset of dysmetabolic diseases. For such purpose, there are many guidelines dictated by scientific research, which propose maximizing the production, decreasing waste and preserving animal wellbeing, through the balanced supply of the nutrient elements.

In order to ensure a correct feeding of the animals, it is necessary that the nutrient elements are uniformly distributed within the mixture of the food product, in a manner such that all the animals take in the provided quantity of each nutrient element.

In order to form a uniform mixture of nutrient elements for animal food products, it is known to use machines, termed in the jargon of the field mixer-wagons, which are adapted to mix the nutrient elements of the food product within a mixing tank thereof and they are commanded to advance along the corridors of the stall in order to distribute the food product within the troughs.

Notwithstanding the use of the abovementioned machines of known type in order to seek to mix, in the most uniform manner possible, the nutrient elements within the food product, in practice non-uniformities are found in the rations of food product that are distributed in the various points of the trough, with consequent assimilation by the animals of inappropriate quantities of nutrient elements.

The aforesaid non-uniformities can be due for example to an incorrect operation of the mixer-wagon, to food product loading errors, or to the change in moisture content of the food product itself, which alters the quantity of dry substance brought by the food product ration.

In particular, the variability of the raw materials which constitute the nutrient elements and the modes of preparation of the food product ration, as well as the use of dedicated machinery such as the aforesaid mixer-wagons, can cause strong differences in the distribution of nutrient elements of the food product, facilitating the separation of the fibrous fractions from those concentrated, preventing a correct simultaneous administration of the nutrient elements to the animals.

Due to the aforesaid non-uniformity of the mixture of the food product, it is necessary to execute controls of the level of uniformity of the nutrient elements in the food product itself.

In particular, analysis processes are known which allow calculating parameters of chemical and physical type, indicative of the concentrations of the nutrient elements of the food product. For example, analysis processes are known for obtaining parameters of chemical type, in particular described by normative texts (CE No. 152/2009 regulation of 27 January 2009; UNI-ISO), by guidelines (IPRA, AOAC method books), by Universities, etc. In addition, analysis processes are known for obtaining parameters of physical type, such as for example the standard process ASAE S424 defined by the American Society of Agricultural Engineers (ASAE), or the process described in the publication of Lammers B.P., Buckmaster D.R., Heinrichs A.J. "A simple method for the analysis of particle size of forage and total mixed rations" Journal of Dairy Science, Number 79, pages 922-928, (1996).

The aforesaid processes of known type allow calculating chemical and physical parameters which permit the analysis of the single sample of the food product, but are unable to describe the overall uniformity of the different food product rations distributed along the entire trough.

For such purpose, further processes are known, like that described in the patent DE 102010033886, which provide for using an infrared spectroscopy for acquiring electromagnetic spectra of portions of the food product and continuously calculating the chemical or physical parameters of the latter. Then, the process provides for comparing the parameters calculated in a specific time instant with those calculated in the preceding instants, for example by calculating

2

means and standard deviations of the aforesaid parameters, in order to determine if the level of uniformity is sufficiently high. In this manner, with the attainment of a suitable uniformity, the stopping of the mixer-wagon is determined as well as the unloading of the food product.

Such processes of known type have in practice shown that they do not lack drawbacks.

The main drawback of known type lies in the fact that in order to obtain the aforesaid chemical and physical parameters by means of spectroscopy, it is necessary to implement a complex calibration algorithm. In particular, different electromagnetic spectra are initially acquired of the components of the food product whose chemical and physical properties are known, and on the basis thereof the aforesaid calibration algorithm is subsequently implemented so as to correlate the electromagnetic spectra with such properties. Consequently, the calibration becomes a very, very long and complex step.

A further drawback lies in the fact that the calculation of the chemical and physical parameters and of the level of uniformity of the mixture requires a high computing cost and consequently requires complex and costly calculation apparatuses.

A further drawback deriving from the requirement of a complex calibration and of a high computation cost lies in the fact that the determination of the uniformity index is a very slow process. Therefore, the level of uniformity at which mixing is to be stopped can be indicated by the calculation apparatus even a long time after the uniformity has been reached, with a consequent loss of efficiency.

Presentation of the invention

The main object of the present invention is therefore that of overcoming the drawbacks manifested by the solutions of known type, by providing a process and an apparatus for mixing a food product for farm animals which allow establishing when to stop the mixing of a food product, in a simple and efficient manner.

Another object of the present invention is to provide a process and an apparatus for mixing a food product for farm animals, which allow establishing when to stop the mixing of a food product with a low computing cost.

Another object of the present invention is to provide a process and an apparatus for mixing a food product for farm animals, which allow establishing when to stop the mixing of a food product without having to determine chemical or physical parameters of the latter.

Another object of the present invention is to provide a process for mixing a food product for farm animals, which is quick to implement.

Another object of the present invention is to provide an apparatus for mixing a food product for farm animals that is entirely functional and reliable in operation, in all use conditions.

Another object of the present invention is to provide an apparatus for mixing a food product for farm animals that is structurally simple and inexpensive to attain.

Brief description of the drawings

The technical characteristics of the invention, according to the aforesaid objects, can be clearly seen from the content of the below-reported claims and the advantages thereof will be more evident from the following detailed description, made with reference to the enclosed drawings, which represent a merely exemplifying and non-limiting embodiment of the invention, in which:

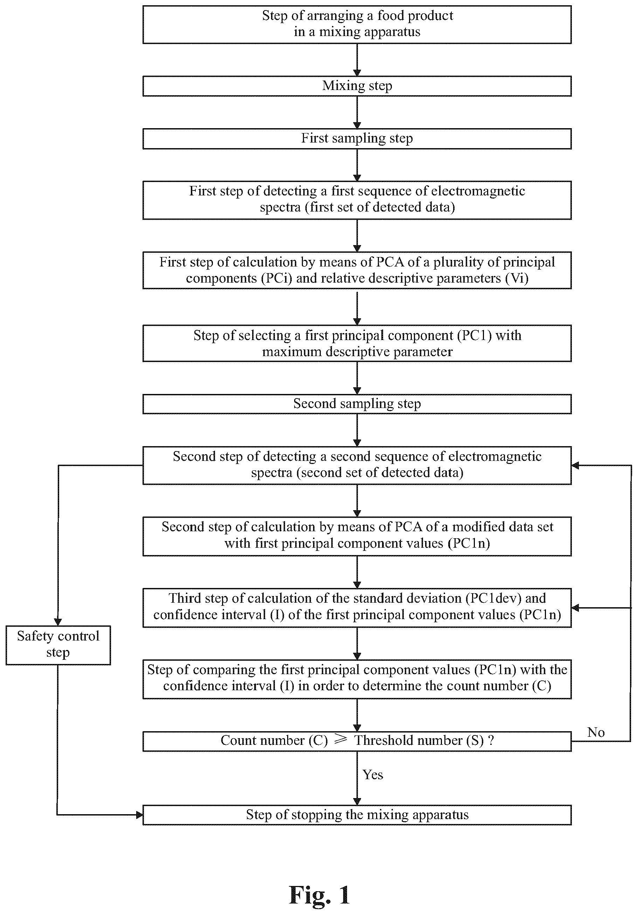

- figure 1 represents a flow diagram relative to one embodiment of the process, object of the present invention;
- figures 2a-n represent a first sequence of electromagnetic spectra of a mixed food product over time, divided into corresponding series by day and farm in which they were detected;
- figures 2o-w represent a second sequence of electromagnetic spectra of a mixed food product over time, divided into corresponding series by day and farm in which they were detected;
- figures 3a-i represent graphs of first principal component values vs. time of the series represented in figures 2o-w, which also illustrate a polynomial trendline and a 95% confidence interval, obtained with several steps of the present process;
- figures 4a-i represent graphs of second principal component values vs. times of the series represented in the figures 2o-w, which also illustrate a polynomial trendline and a 95% confidence interval, obtained with several steps of the present process;
- in figures 5a-i, three-dimensional graphs are represented comprising the values in figures 3a-i and 4a-i and in which also a fitting plane is represented;
- figures 6a-i represent graphs of the values of figures 3a-i in which the aforesaid values have been modified with correction methods;
- figures 7a-i represent graphs of the values of figures 4a-i in which the aforesaid values have been modified with correction methods;

- figures 8a-i represent graphs of the values of figures 5a-i in which the aforesaid values have been treated with correction methods;
- figures 9a-i represent graphs of standard deviations of the values in figures 6a-i vs. tempo, which also illustrate a polynomial trendline and a 95% confidence interval;
- figures 10a-i represent graphs of standard deviations of the values in figures 7a-i vs. tempo, which also illustrate a polynomial trendline and a 95% confidence interval.

Detailed description of a preferred embodiment

The present process and apparatus are advantageously intended to be employed in intensive animal farming in order to execute an analysis of uniformity of a food product, preferably before distributing it to farm animals, such as beef or milk cattle, pigs, goats, sheep, buffalo, etcetera.

Such intensive (or industrial) animal farming is generally placed in stalls normally provided with one or more corridors, along the sides of which the animal food troughs are placed.

In particular, the food product intended to be distributed to the animals (termed in the technical jargon of the field unifeed or Total Mixed Ratio (TMR)) normally comprises a mixture of solid bodies (in the form for example of ground vegetables and/or grains) of multiple nutrient elements. In particular, the mixture of the food product comprises forage (such as corn, sorghum, etc.) and concentrated foods which for example contain protein components (such as soy grains) and/or energy components (such as corn grains).

According to the invention, the process comprises a step of arranging a food product to be mixed within an apparatus for mixing. In particular, such step advantageously provides for the arrangement of an apparatus for mixing and, subsequently, the loading of one or more doses of solid bodies aimed to be mixed in order to constitute the food product.

More in detail, the apparatus for mixing comprises a mixing tank in which the food product to be mixed is intended to be placed and mixing means, for example one or more screws, placed within the mixing tank and actuatable in order to mix the food product. In particular, in the loading step, the solid bodies are preferably loaded within the mixing tank.

Advantageously, the apparatus for mixing comprises a load arm intended to load the solid bodies in the mixing tank.

Of course, without departing from the protective scope of the present invention, the apparatus for mixing might not comprise a load arm and the loading of the food product can also be executed manually or by means of a separate loading apparatus.

The present process also comprises a mixing step, in which the apparatus for mixing is actuated in order to mix the food product. In particular, in the mixing step, the mixing means of the apparatus for mixing are actuated.

Advantageously, the present process comprises a step of distribution of the food product in the trough along a front of the trough itself.

With the term "trough front" it is intended the physical space in which the food product is distributed and is in particular constituted by a corridor, placed in front of the trough positions, which covers a depth equal to that actually occupied by the distributed food product, e.g. about a meter.

Advantageously, the food product can be manually distributed or distributed by means of the use of the same apparatus for mixing, which can for example be integrated in a mixer-wagon. In particular, the latter is actuated to advance in the corridor of the stall in order to distribute the food product along the front of the trough.

The present method also comprises a first step of detecting a first sequence of electromagnetic spectra reflected by the food product. In particular, the first detection step leads to the acquisition of a first set of detected data characteristic of the electromagnetic spectra of the first sequence. Such first set of detected data comprises different variables, such as for example wavelengths and/or wavenumbers and/or reflectance of the measured electromagnetic spectra.

Preferably, the aforesaid reflection electromagnetic spectra are electromagnetic spectra in the near infrared (NIR) wavelengths.

In accordance with a particularly advantageous embodiment of the present invention, the aforesaid electromagnetic spectra are obtained by means of a spectrometer, preferably by means of a NIR spectrometer operating in the near infrared spectrum, suitably adjusted and calibrated.

In particular, the employed spectrometer is the device termed poliSPEC NIR (by IT Photonics with registered office in Breganze (VI) Italy), self-power supplied with battery, with cooled InGaAs PDA sensor, spectral band 900-1700 nm, optical resolution 2 nm, with integrating sphere.

Advantageously, the electromagnetic spectra of the first sequence are detected in consecutive and preferably equidistant time instants.

Advantageously, the electromagnetic spectra of the first sequence are detected on corresponding first samples of the food product and in particular the process comprises a first step of sampling the food product, in which multiple first samples corresponding to food product portions are identified. Advantageously, the first samples of the food product are identified within the mixing apparatus, more particularly in the mixing tank, preferably during the mixing of the food product.

In combination, or as an alternative, the first samples of food product are identified during and/or after the distribution step, in particular along the trough front.

More in detail, in accordance with a first embodiment variant, such first samples can be identified in the mixing apparatus at the same point at different time intervals. In such case, in the first detection step, the spectrometer is pointed towards a defined area of the mixing tank and measures the electromagnetic spectra of a detection window through which the food product flows during the mixing.

In accordance with a second embodiment variant, such first samples can be in the mixing apparatus at different points of the mixing tank. In such case, in the first detection step the spectrometer is pointed towards different areas of the mixing tank, or multiple spectrometers are present that are pointed towards the aforesaid different areas. In this manner, the detection of the electromagnetic spectra can also occur in the event in which the food product is not yet mixed and hence is not moved.

In accordance with a third embodiment variant, such first samples can be distributed in corresponding different positions that are spaced from each other along the front of the trough. In such case, the sampling step can be advantageously executed on the food product ration already deposited in the trough or on the ration of the food product before it is distributed, for example on the food product batch exiting from the mixer-wagon. In the first detection step, the spectrometer is pointed towards different areas of the trough, or it is pointed towards a defined area of an unloading section of the mixing tank and measures the electromagnetic spectra of a detection window through the exiting food product flows.

Of course, without departing from the protective scope of the present invention as defined in the claims, the aforesaid embodiment variants can be provided as alternatives or in combination with each other.

In accordance with the present invention, the process comprises a first calculation step, in which the first set of detected data is subjected to Principal Component Analysis (PCA) in order to obtain a plurality of principal components PCi. In particular, each principal component PCi has, associated therewith, a corresponding descriptive parameter Vi of the variability percentage of the first set of detected data.

In particular, the Principal Component Analysis (abbreviated PCA) is also known with the name Karhunen-Loève transform and is a technique of known type for simplifying the data used in the scope of the multivariate statistics. Advantageously, in the first calculation step, a first part of the Principal Component Analysis is applied which provides for transforming the variables of the first set of detected data into principal components PCi.

More in detail, the first calculation step comprises a first standardization stage for obtaining a first standardized data set. In the first standardization stage, all the variables are advantageously transformed into standardized variables which vary in the same interval. In this manner, one prevents that, due to great differences between the intervals of the variables, the variables with larger intervals dominate over those with smaller intervals (for example, a variable comprised between 0 and 100 would tend to dominate over a variable comprised between 0 and 1, if not suitably standardized).

Mathematically, in the first standardization stage, the mean of the values is advantageously subtracted from each value of each variable, and it is divided by their standard deviation.

The first calculation step also comprises advantageously a correlation stage, in which it is determined if the variables (in particular the standardized variables) are correlated with each other. In particular, in the correlation stage, the covariance matrix of the first set of detected data and preferably of the first standardized data set is calculated.

With "covariance matrix" it will be intended a symmetrical matrix that has as entries the covariances associated with all the possible pairs of the variables (in particular of the standardized variables). More in detail, if the sign of the covariance of a pair of variables is positive, then the two variables increase or decrease together (correlated), if negative then one increases when the other decreases (inversely correlated).

Advantageously, the first calculation step comprises a stage of calculation of the eigenvalues and of the corresponding eigenvectors of the aforesaid covariance matrix. In particular, the eigenvalues and the eigenvectors are of a number equal to the variables of the first set of detected data (and standardized). More in detail, the eigenvectors of the covariance matrix are algebraically the directions of the axes where there is more variance (i.e. more information) and are named principal components PCi. In particular, the principal components PCi are less interpretable and do not have any actual significance since they are constructed as linear combinations of the initial variables. Advantageously, the eigenvalues are the coefficients corresponding to the eigenvectors and represent the total variance corresponding to each principal component PCi.

Advantageously, the calculation step comprises a step of ordering the eigenvectors with corresponding eigenvalues, in particular from the highest to the lowest, in order to obtain the principal components in order of significance.

After having identified the principal components PCi, the first calculation step provides for a stage of determination of the descriptive parameter Vi of the variability percentage of the first set of detected data, i.e. the variance percentage (information) represented by each principal component PCi. Advantageously, the aforesaid descriptive parameter Vi is calculated by dividing the eigenvalue of each principal component PCi by the sum of the eigenvalues.

Therefore, by calculating the eigenvectors and ordering them by their eigenvalues in decreasing order, this advantageously allows finding the principal components PCi in order of significance.

Given that the Principal Component Analysis is an analysis of known type, the aforesaid stages will not be further described hereinbelow. Of course, the above-reported stages are indicated as a non-limiting example and the Principal Component Analysis can be carried out in a different manner.

In accordance with the idea underlying the present invention, the process also comprises a selection step, in which from among the plurality of principal components PCi, at least one first principal component PC1 is selected that is associated with the descriptive parameter Vi with maximum value.

Preferably, in the selection step, the principal components PCi are selected with a descriptive parameter Vi greater than 5%, preferably greater than 10%.

A second principal component PC2 and/or a third principal component PC3 can also be selected, up to the i-th principal component PCi, respectively associated with second, third, ..., i-th descriptive parameter Vi in decreasing order.

In this manner, it is possible to select the principal components to be maintained, discarding those of little significance (with low eigenvalues).

In addition, it is advantageously possible to simplify the study of the variability of the food product during the mixing without having to explicitly take under consideration its chemical and physical characteristics, facilitating the calculation.

The present process also comprises a second step of detecting a second sequence of electromagnetic spectra reflected by the food product. In particular, the second detection step leads to the acquisition of a second set of detected data characteristic of the electromagnetic spectra of the aforesaid second sequence. Such second set of detected data comprises variables, such as for example wavelengths and/or wavenumbers and/or reflectance of the measured electromagnetic spectra. In particular, the variables of the second sequence of electromagnetic spectra are equivalent to the variables of the first sequence of electromagnetic spectra.

Preferably, the aforesaid reflection electromagnetic spectra are electromagnetic spectra in the near infrared (NIR) wavelengths.

In accordance with a particularly advantageous embodiment of the present invention, the aforesaid electromagnetic spectra are obtained by means of a spectrometer, preferably by means of a NIR spectrometer operating in the near infrared spectrum, suitably adjusted and calibrated. In particular, the spectrometer employed is the same that is involved in the detection in the first detection step. Advantageously, the electromagnetic spectra of the second sequence are detected in consecutive and preferably equidistant time instants. Advantageously, in addition, the second detection step is executed continuously during the mixing of the food product.

In particular, the electromagnetic spectra of the second sequence are detected on corresponding second samples of the food product and in particular the process comprises a second step of sampling the food product, in which multiple second samples corresponding to food product portions are identified. Advantageously, the second samples of the food product are identified within the mixing apparatus, more particularly in the mixing tank, preferably during the mixing of the food product.

In combination or as an alternative, the second samples of food product are identified during and/or after the distribution step, in particular along the trough front.

Advantageously, the second detection step and/or the second sampling step are executed analogously to that described for the first detection step and the first sampling step and therefore will not be further described in detail hereinbelow.

Advantageously, the second detection step is executed after the first detection step. In this manner, the electromagnetic spectra of the first sequence are used for calibration, while the electromagnetic spectra of the second sequence are used for the testing that will be described hereinbelow. The first and the second sequence will therefore be advantageously different from each other.

Of course, without departing from the protective scope of the present invention, the second sequence of electromagnetic spectra can entirely or partly coincide with the first sequence of electromagnetic spectra. In this case, the second detection step will be intended as simultaneous and/or coinciding with the first detection step.

The process according to the invention also comprises a second calculation step, in which the second set of detected data is at least partially transformed into a second modified data set by means of Principal Component Analysis (PCA) on the basis of the selected first principal component PC1. In this manner, a second modified data set is defined, comprising a plurality of first principal component values PC1n each associated with a corresponding electromagnetic spectrum of the second sequence.

More in detail, the second calculation step comprises a stage of generation of a transformation matrix formed at least with the first principal component PC1 and preferably with all the principal components PCi selected in the selection step. In particular, the transformation matrix is a matrix that has as columns the eigenvectors of the selected principal components PCi.

Preferably, the second calculation step comprises a second standardization stage in which the second set of detected data is standardized, in a manner analogous to that described above in the first standardization stage.

Advantageously, the second calculation step comprises a stage of transformation of the second set of detected data (preferably of the second standardized data set) into a modified data set by means of multiplication of the transposed transformation matrix (i.e. of the transposed matrix of the transformation matrix) with the second transposed detected data set (i.e. the transposed matrix of the second set of detected data). Alternatively, the modified data set in the transformation stage can be obtained by executing the transposition of the product between the second set of detected data and the transformation matrix.

Also in this case, given that the Principal Component Analysis is an analysis of known type, the aforesaid stages will not be further described hereinbelow. Of course, the above-reported stages are indicated as an example and the Principal Component Analysis can be carried out in a different manner.

In addition, the process can provide for further steps or stages of data processing in order to carry out, in a known manner, noise reductions, corrections, selections or cleaning of the sets of detected data.

According to the invention, the process also comprises a third calculation step, in which the standard deviation PC1dev is calculated of at least one set of first principal component values PC1n of the plurality of first principal component values PC1n and, on the basis of the standard deviation PC1dev, a confidence interval I is calculated.

With the expression "confidence interval", it will be intended an interval whose ends are obtained by summing and subtracting a margin of error E from the mean of the first principal component values PC1n. In particular, in the third calculation step, the mean of the set of first principal component values PC1n is initially calculated, which is advantageously also used for the calculation of the standard deviation PC1dev. Subsequently, the margin of error E is advantageously calculated with the following formula:

$$E = Z_{\alpha/2} \cdot \frac{PC1dev}{\sqrt{(n)}}$$

in which $Z_{\alpha/2}$ is a confidence coefficient dependent on a confidence percentage $\alpha$, PC1dev is the standard deviation and n is the number of first principal component values PC1n in the set of first principal component values PC1n. In particular, the confidence percentage $\alpha$ is greater than or equal to 80%, preferably greater than or equal to 90%. In addition, $Z_{\alpha/2}$ advantageously represents the critical value of the normal standard distribution of the data and is calculated by means of suitable table of known type.

In accordance with a preferred embodiment of the invention, in the third calculation step the second sequence of electromagnetic spectra is divided into a plurality of sub-sequences, each corresponding to a different set of first principal component values PC1n. Therefore, the standard deviation PC1dev is calculated for the first principal component values PC1n of each set of first principal component values PC1n. In addition, the confidence interval I is advantageously established on the basis of each standard deviation PC1dev associated with the corresponding set of first principal component values PC1n. According to the invention, the process also comprises a step of comparing the set of first principal component values PC1n with the confidence interval I in order to determine the count number C of the first principal component values PC1n falling within the confidence interval I.

In accordance with the preferred embodiment of the invention, in the comparison step the count number C is determined for each corresponding confidence interval I of each set of first principal component values PC1n.

The process also comprises a step of stopping the mixing of the apparatus for mixing following at least the attainment, preferably the exceeding, of a pre-established threshold number S by the aforesaid count number C.

In this manner, it is advantageously possible to establish the uniformity of the mixture in a simple manner and with low computing cost, simply from the observation of the progression at least of the first principal component PC1n (and if necessary also of the subsequent) associated with the collected data set. It is therefore advantageously possible to exclude the complex calculation algorithms of the food product parameters on the basis of the collected electromagnetic spectra, since the statistical processing of the data is sufficient.

In accordance with the preferred embodiment of the invention, in the stopping step, the mixing is stopped upon attainment of a minimum limit of sets of first principal component values PC1n, preferably one after the other, having the count number C greater than or equal to the pre-established threshold number S. Advantageously, in the decision step, the number of pre-established threshold S is equal to the number of electromagnetic spectra of the corresponding sub-sequence and is preferably comprised between 11 and 61, still more preferably between 21 and 51.

Preferably, the process also comprises a step of safety control, at least partially executed simultaneously with the second detection step, in which the number of electromagnetic spectra of the second sequence is counted, in which the apparatus for mixing is stopped if the number of electromagnetic spectra exceeds a preset maximum safety value.

Preferably, the maximum safety value is comprised between 800 and 1400 measurements, preferably between 900 and 1300, still more preferably between 1000 and 1200.

In combination or as an alternative, in the control step, a value of physically effective neutral detergent fiber (peNDF) of the food product is measured and the apparatus for mixing is stopped if such value of physically effective neutral detergent fiber (peNDF) falls within an ideal fiber interval, in a manner such that the mixing does not bring the quantity of physically effective neutral detergent fiber (peNDF) to an overly high or overly low value.

In this manner, in the event in which the required uniformity is not obtained, it is still advantageously possible to stop the mixing without compromising the food product.

Reported hereinbelow is one embodiment of the invention executed as application study on a plurality of samples collected in three different farms (hereinbelow and in the figures named "Farm 1", "Farm 2" and "Farm 3").

In particular, the first and second detection step were executed by using a mixing apparatus integrated on a mixer-wagon, and on different control days an overall data set was collected (comprising the detected first data set and the detected second data set described hereinbelow) composed of about 128,478 electromagnetic spectra, and comprises NIR electromagnetic spectra detected during the loading, the mixing and the distribution of the food product. The electromagnetic spectra were detected by means of the spectrometer named poliSPEC NIR, described above.

The arranging step, the mixing step and the first and second detection step of the process have therefore been executed in this case substantially together, while all the other steps (in particular the calculation and selection steps) were subsequently executed.

For this study, only the electromagnetic spectra collected during the distribution of the food product - executed simultaneously with the mixing - were selected and used, i.e. 19530 spectra, and after a more accurate selection 12551 electromagnetic spectra.

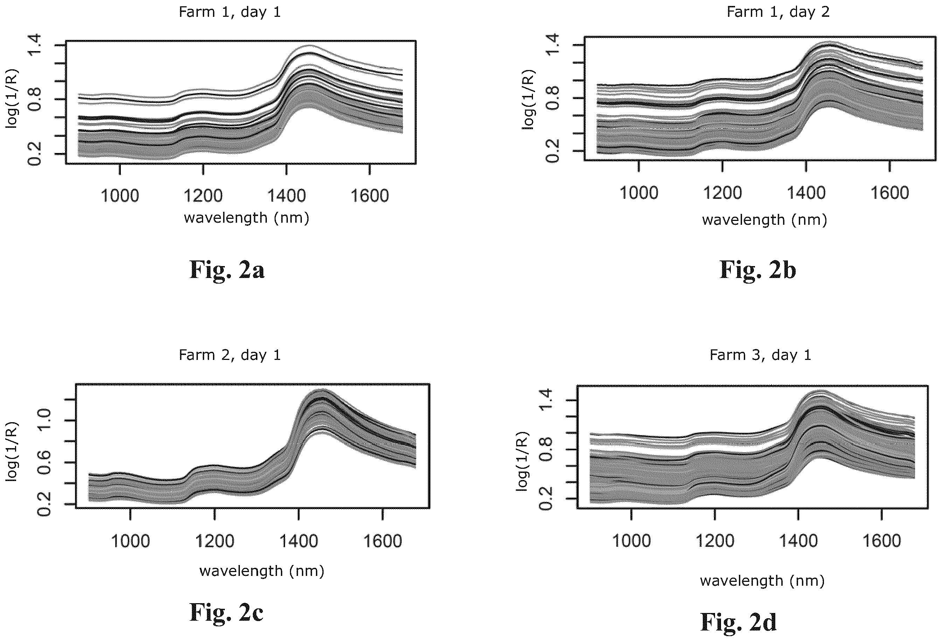

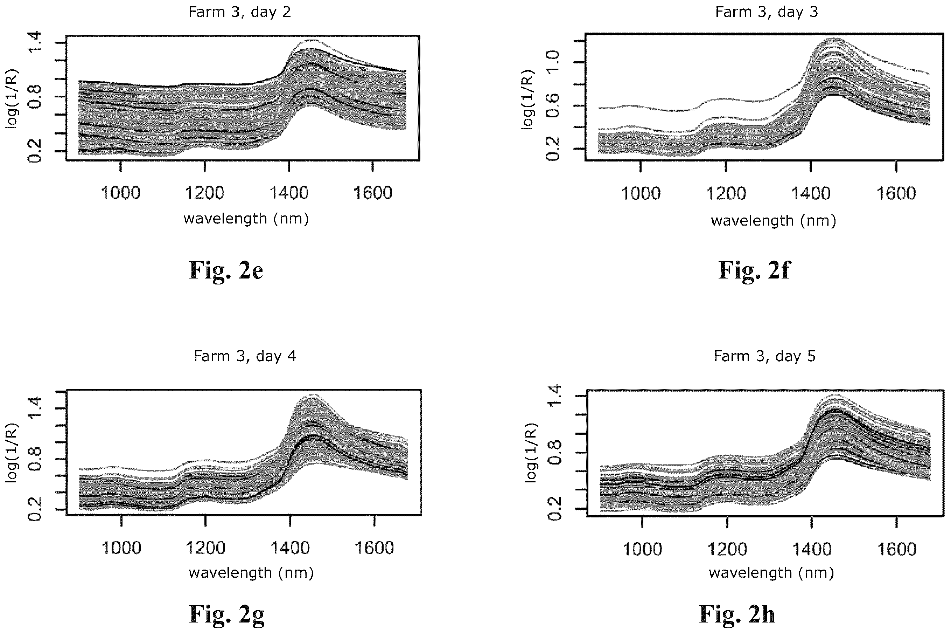

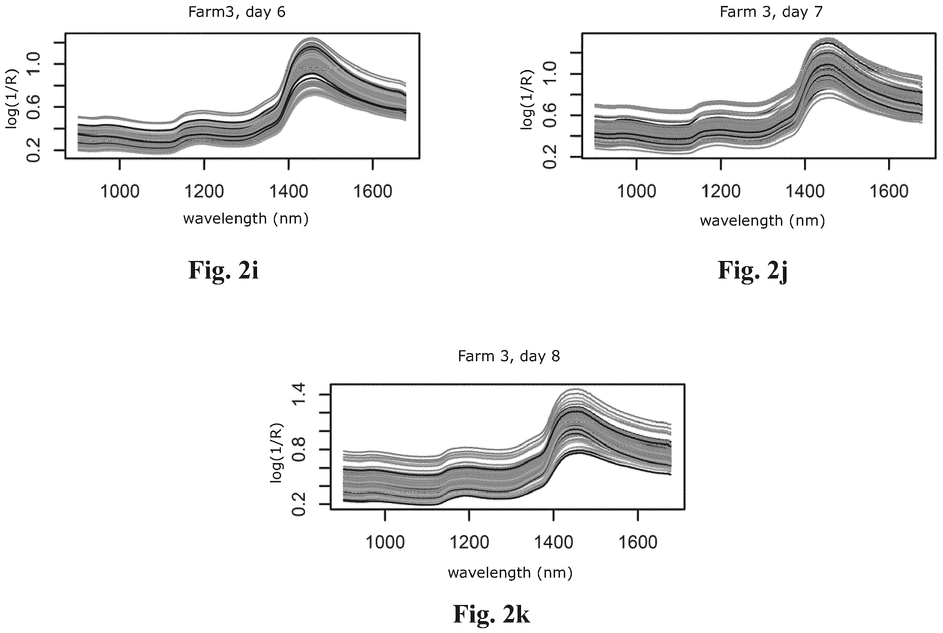

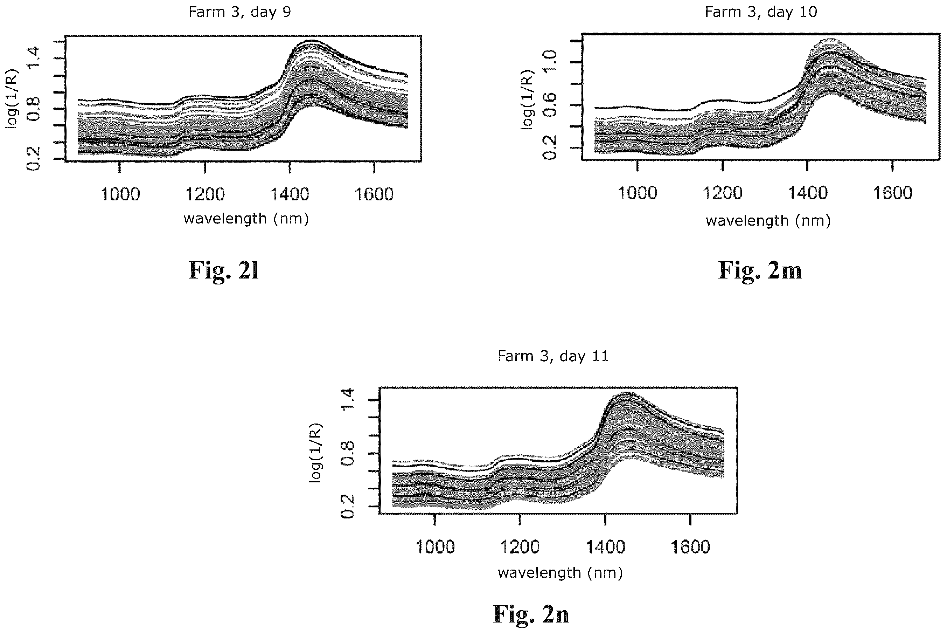

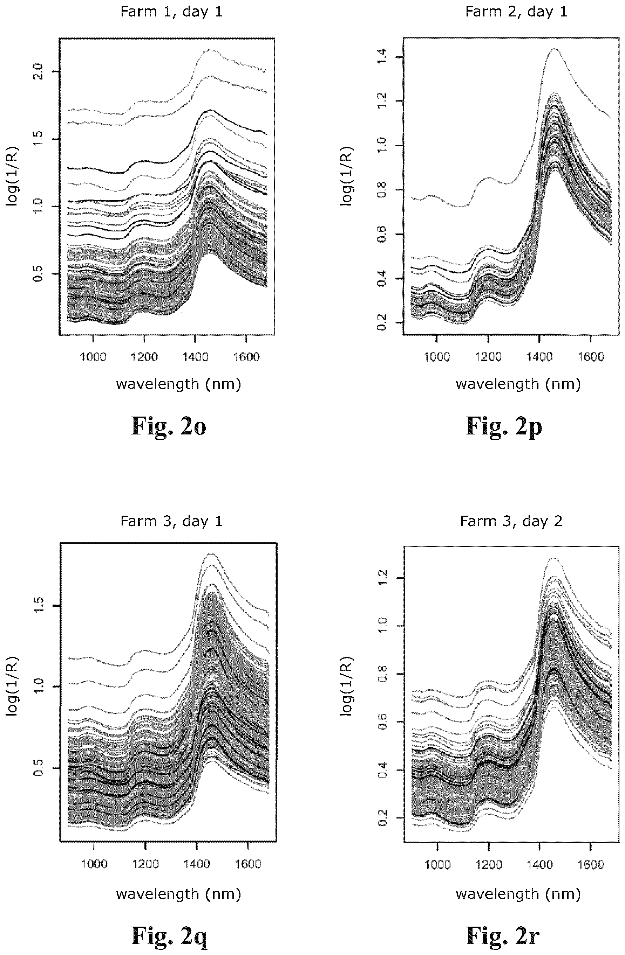

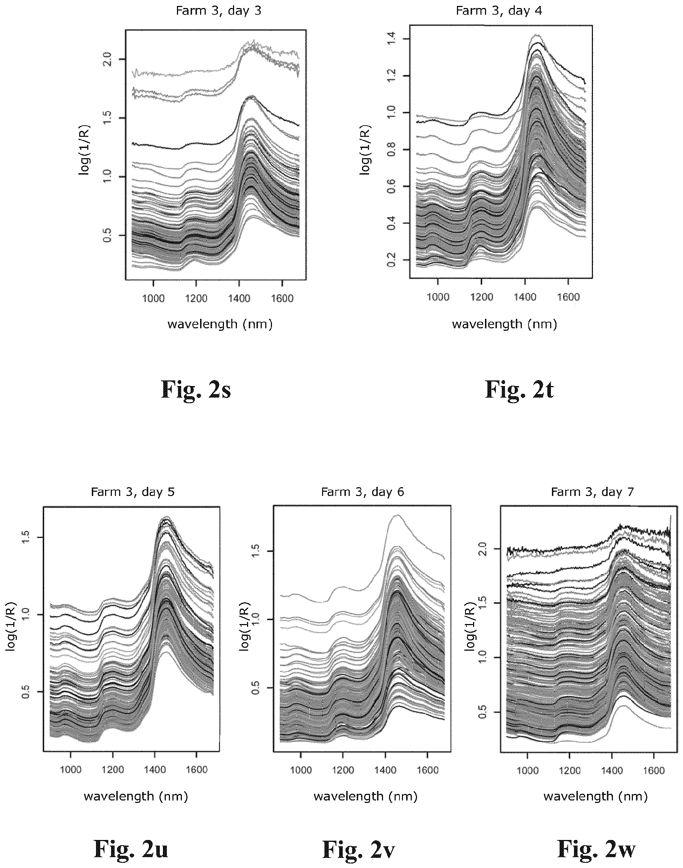

The overall data set was divided into different series by farm and by control day and the electromagnetic spectra of corresponding series are illustrated in figures 2a-w.

In addition, the overall data set was divided into a first set of detected data, also termed "training dataset", comprising 7,411 electromagnetic spectra, and a second set of detected data, also termed "testing dataset", comprising 5,140 electromagnetic spectra, seeking to maintain the same representativeness of the farms. Subsequently, the first calculation step was executed in which the first set of detected data has been subjected to the standardization stage by means of a method known as "Standard Normal Variate" and subsequently subjected to a detrending correction stage with first derivative by means of Savitzky-Golay method, using second-degree polynomials applied with window size equal to 11 (i.e. considering a set of 11 points at a time), obtaining a first standardized (and correct) data set.

In the first calculation step, the above-described stages were also executed in order to apply the Principal Component Analysis (PCA) to the first standardized data set and the first ten principal components $PC_i$ were obtained as well as the corresponding descriptive parameters $V_i$, reported in Table 1.

**Table 1 - Principal components PCi obtained and descriptive parameters Vi**

| PCi | PC1 | PC2 | PC3 | PC4 | PC5 | PC6 | PC7 | PC8 | PC9 | PC10 |
|---|---|---|---|---|---|---|---|---|---|---|
| Vi (%) | 73.3 | 20.8 | 2.4 | 1.2 | 0.7 | 0.4 | 0.3 | 0.1 | 0.1 | 0.1 |

Hereinbelow, in the selection step, a first principal component PC1 and a second principal component PC2 were selected which have the descriptive parameters $V_i$ with the higher values.

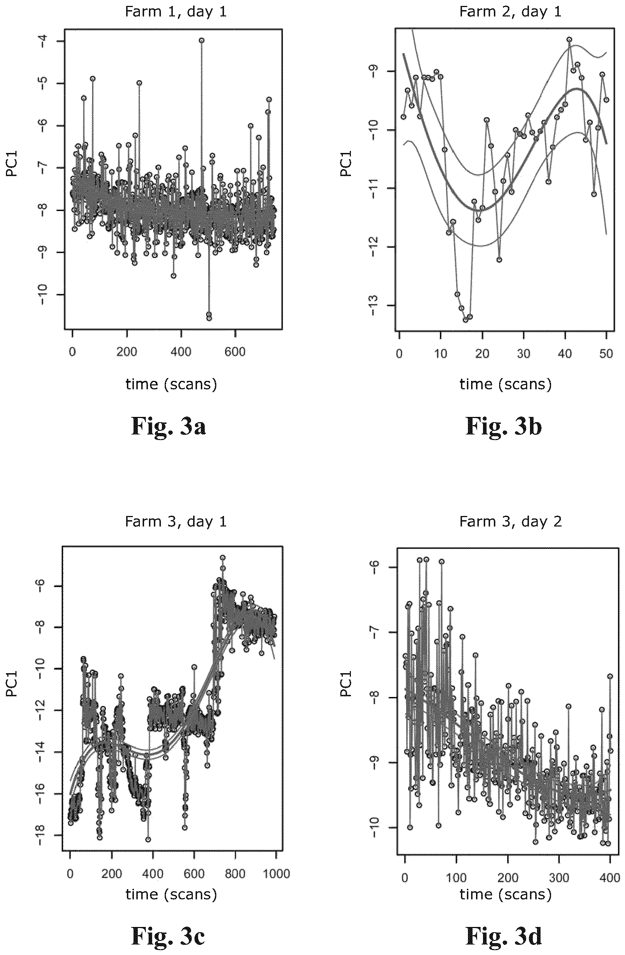

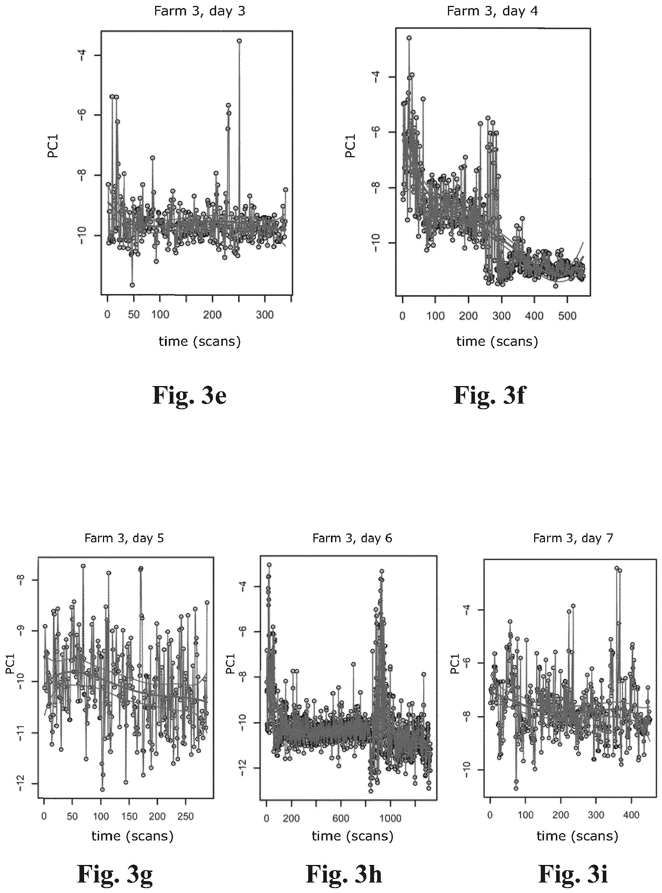

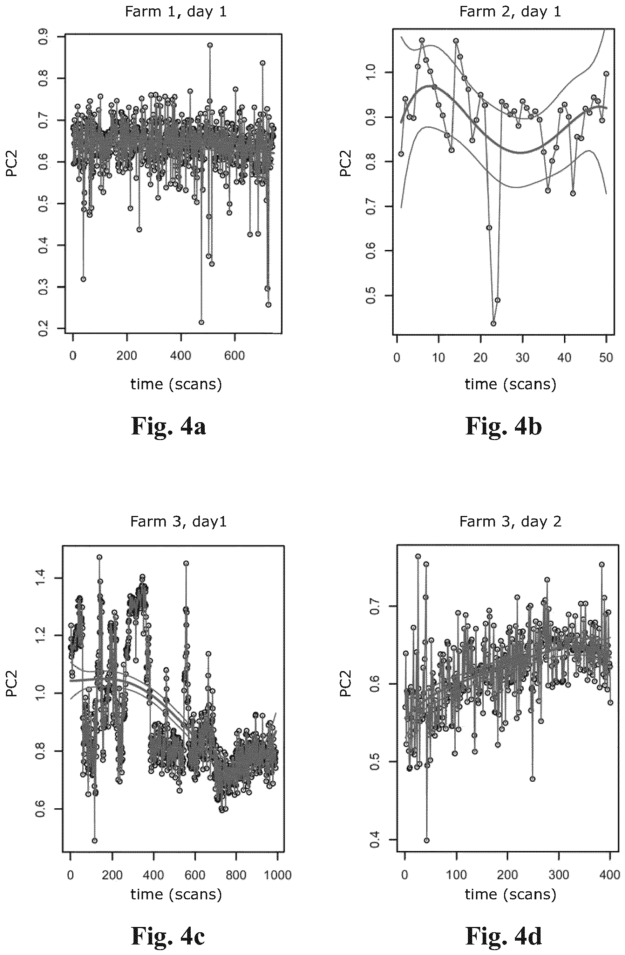

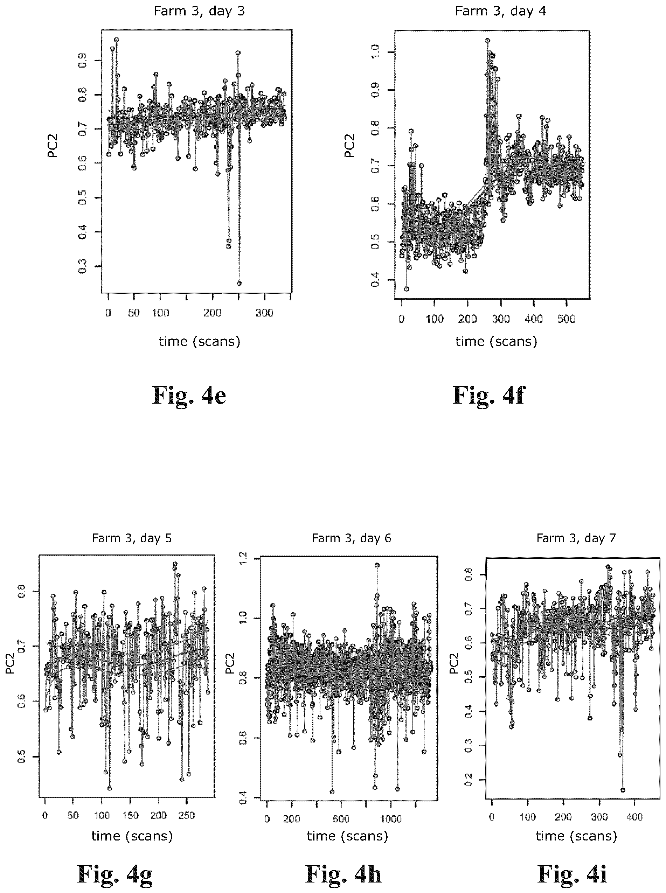

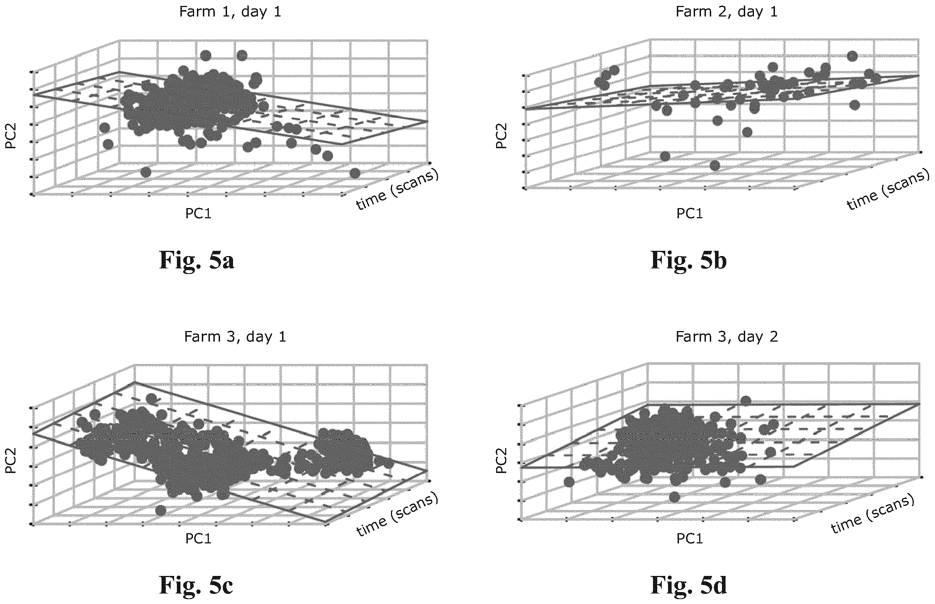

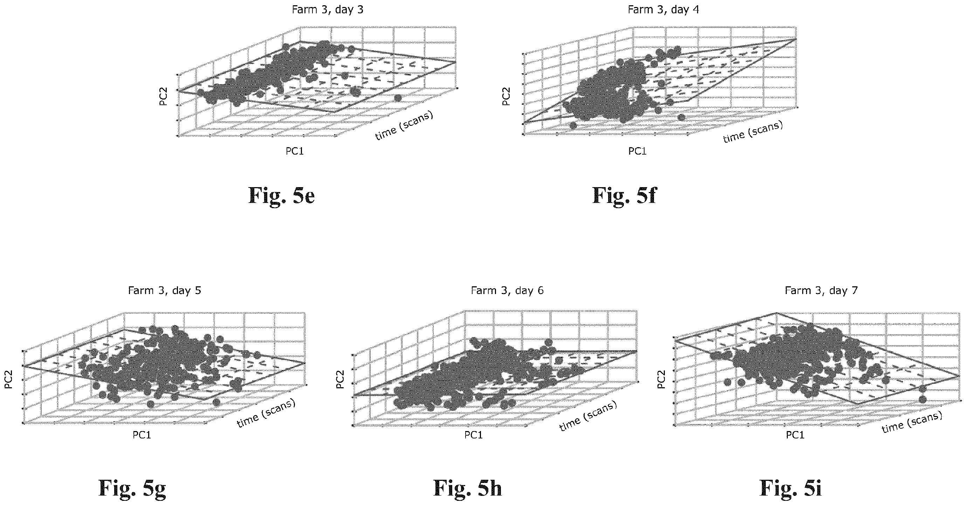

The second calculation step was then executed in which the Principal Component Analysis (PCA) has been applied to the second set of detected data on the basis of the first and of the second principal components PC1, PC2 in order to obtain a modified data set comprising first principal component values PC1n and second principal component values PC2n for each electromagnetic spectrum. The graphs of the first principal component and second principal component values PC1n, PC2n vs. time are illustrated in figures 3a-i and 4a-i, which also illustrates a polynomial trendline and the 95% confidence interval I and in figures 5a-i, which also illustrates a fitting plane 6rt.

In addition, a correction was executed, in which the first and second principal component values PC1n, PC2n that do not fall within a confidence interval I with confidence percentage equal to 99% have been discarded from the modified data set.

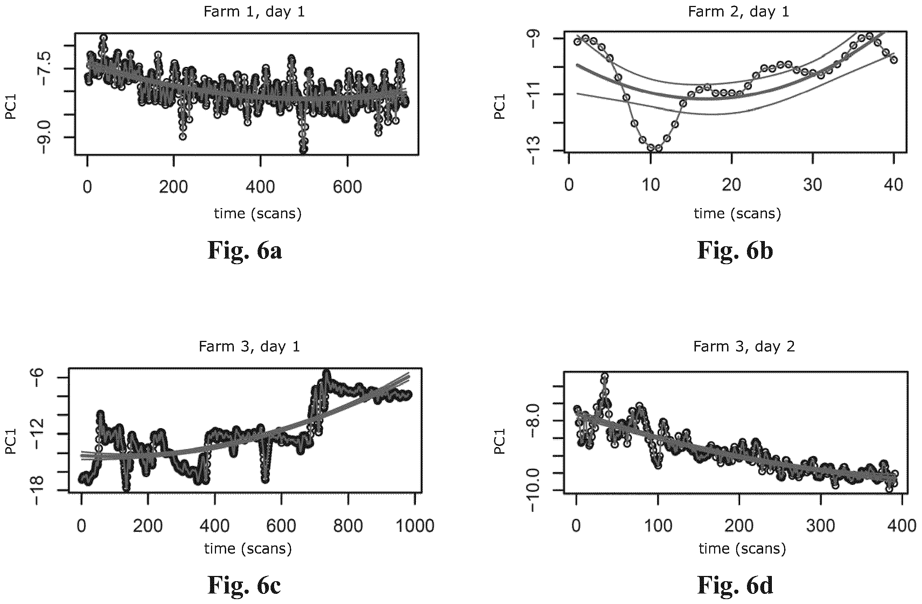

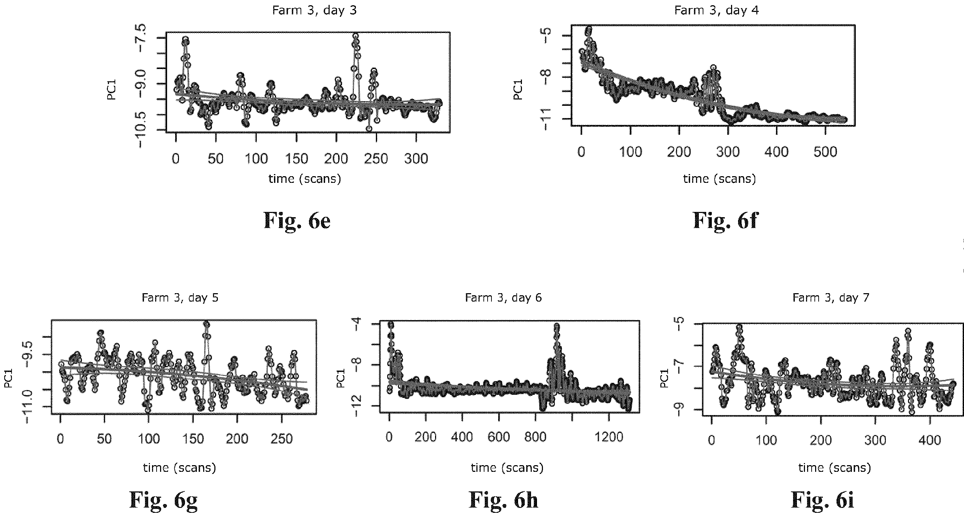

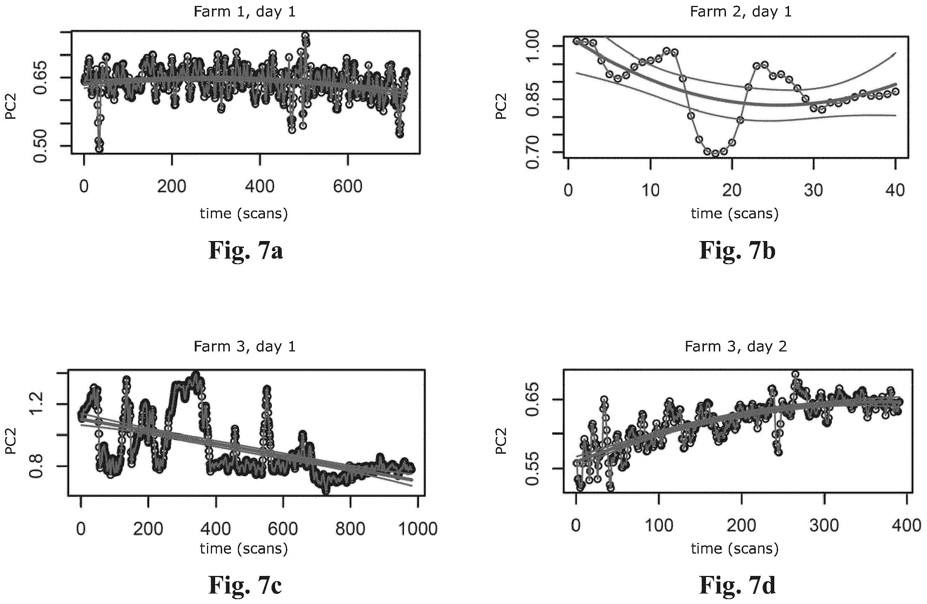

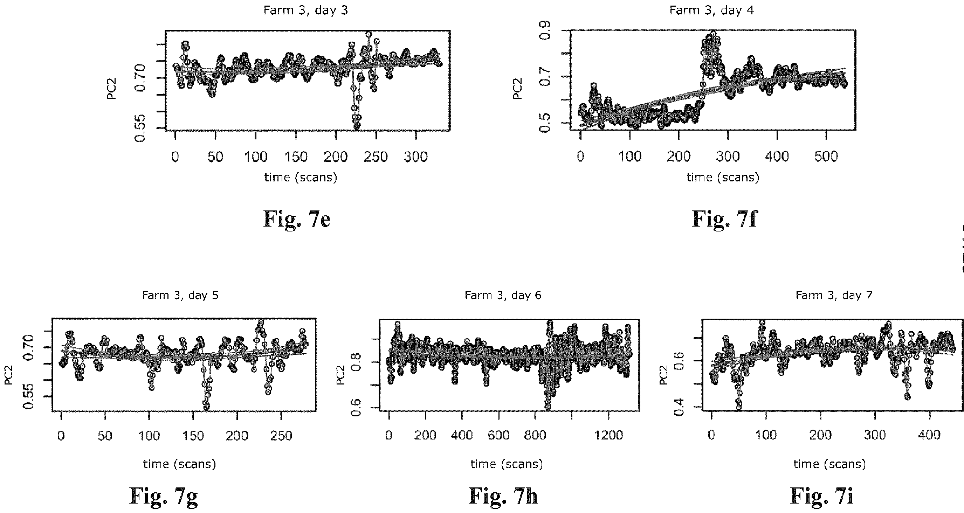

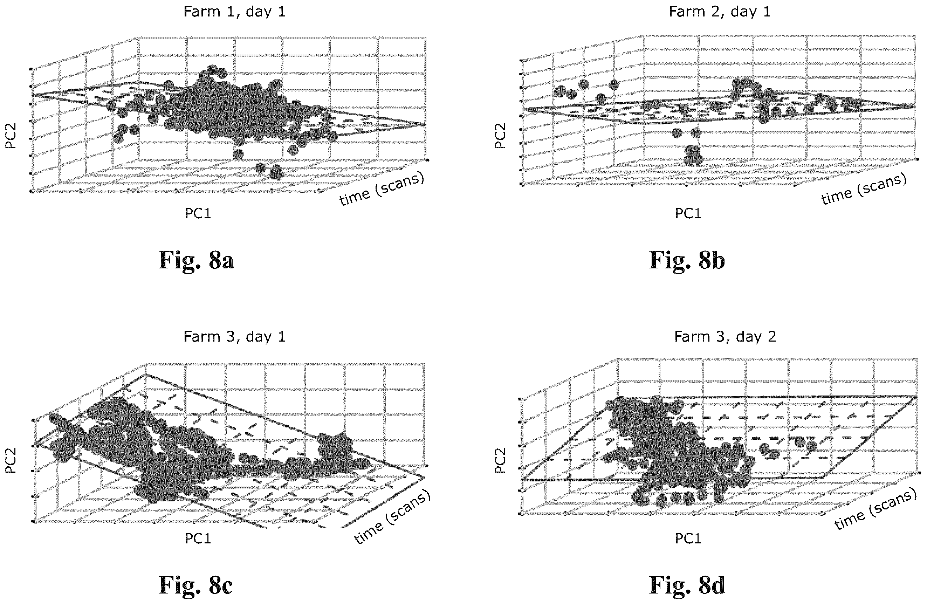

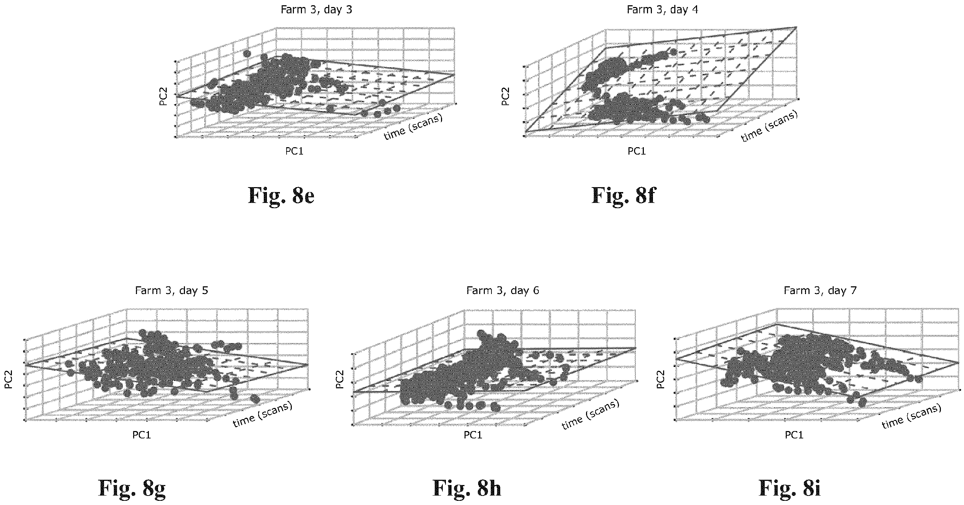

It is noted in the figures that a unique progression explicative of the mixture degree cannot be properly observed. Hence a further correction has been applied by means of Savitzky-Golay method at the first principal component and second principal component PC1n, PC2nm values using a differentiation order equal to zero, a second-degree polynomial and a window size of 11. The graphs of the first principal component and second principal component values PC1n, PC2n thus modified vs. time are illustrated in figures 6a-i and 7a-i, which also illustrates a polynomial trendline and the 95% confidence interval I in figures 8a-i, which also illustrates a fitting plane.

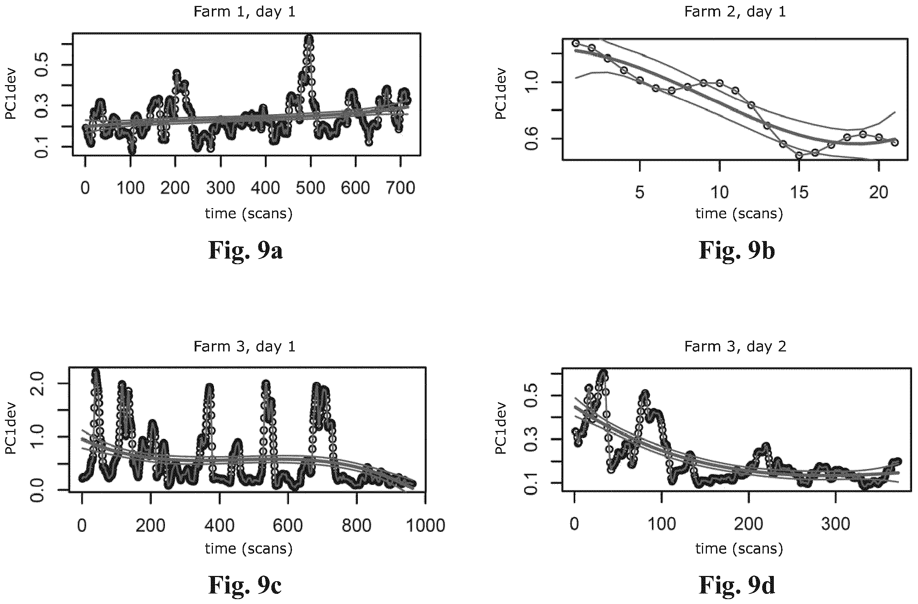

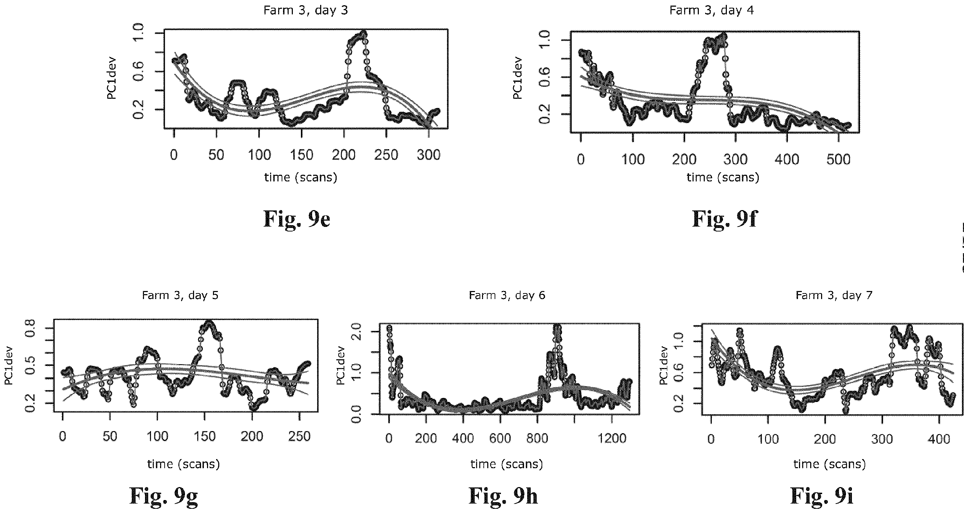

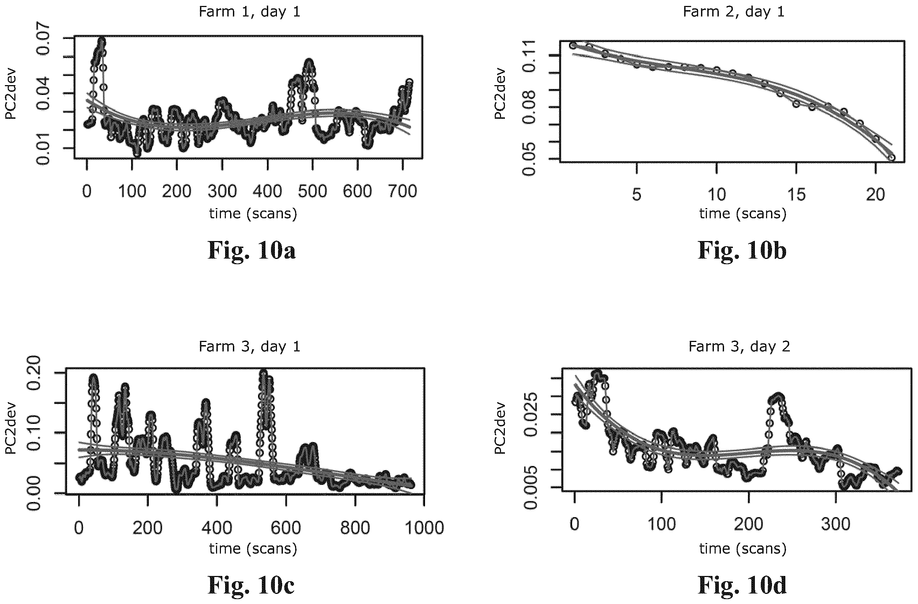

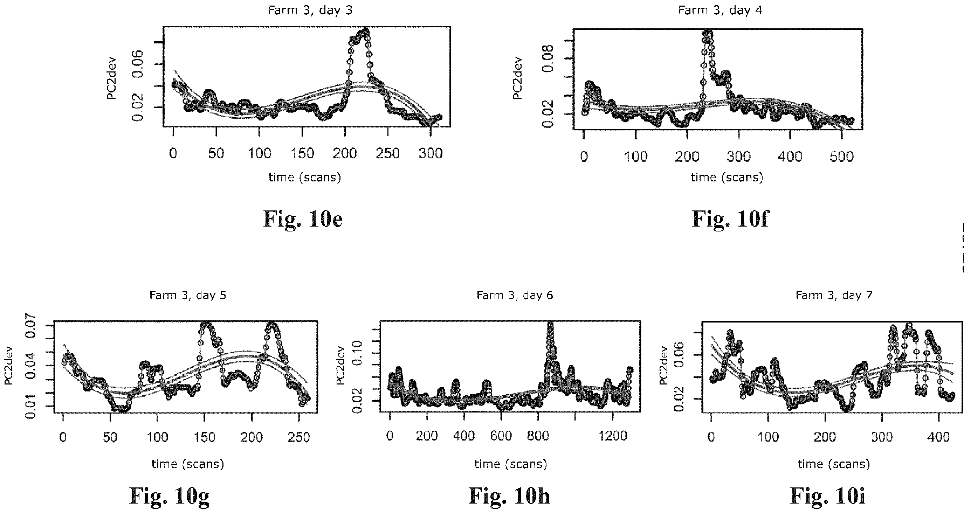

Subsequently, the third calculation step has been executed in which the standard deviation PC1dev, PC2dev was calculated for sets of first and second principal component values PC1n, PC2n, each comprising 21 electromagnetic spectra detected in sequence. The graphs of the standard deviations PC1dev, PC2dev thus obtained are illustrated in figures 9a-i and 10a-i, which also illustrates polynomial trendline and the confidence interval I of the 95% standard deviations.

As can be observed from the obtained data, the duration of the mixture is very different between and within farms, hence not very uniform. For example, the mixtures of farm 1 vary from a minimum of 250 to a maximum of 1000 scans (detections of electromagnetic spectra). Where discontinuities are visible in the graphs (in particular in the graphs relative to the spectra acquired on days 1 and 4 of Farm 3), it is possible to assume that there have been interruptions of the mixing.

It is advantageously observed that in a period comprised between 400-600 scans (excluding those indicated above), there is substantially a stabilization of the first and second principal component values PC1n, PC2n - see figures 6a-i and 7a-i on the matter.

Therefore, the aforesaid scan value can be considered as an indication of the minimum threshold of mixing for obtaining the uniformity of the mixture. By analyzing the data with the above-described process, and preferably also the polynomial regression curve, it is instead possible to determine the optimal point of the mixture.

Also constituting the object of the present invention is an apparatus for mixing a food product for farm animals, and such apparatus in particular is employable for executing the above-described process in an automated manner.

The apparatus, object of the present invention, comprises a mixing tank in which a food product to be mixed is intended to be placed and mixing means placed within said mixing tank and actuatable in order to mix said food product.

Advantageously, the apparatus also comprises a load arm intended to load the solid bodies which constitute the food product in the mixing tank.

Of course, without departing from the protective scope of the present invention, the apparatus for mixing also might not comprise a load arm and the loading of the food product can also be executed manually or by means of a separate loading apparatus.

The apparatus also comprises a detection device arranged for detecting at least one first and a second sequence of electromagnetic spectra reflected by the food product and acquiring a first and a second set of detected data characteristic of the electromagnetic spectra respectively of the first and second sequence. Advantageously, the detection device comprises a NIR spectrometer operating in the near infrared spectrum. In particular, the spectrometer is constituted by the device named poliSPEC NIR (by IT Photonics with registered office at Breganze (VI) Italy), self-power supplied with battery, with cooled InGaAs PDA sensor, spectral band of 900-1700 nm, optical resolution 2 nm, with integrating sphere. Advantageously, the spectrometer comprises, in a manner per se known to the man skilled in the art, at least one transmitter arranged for emitting at least one beam of radiations, in particular with spectral band 900-1700 nm, intended to hit the analyzed sample of food product, and an optical receiver arranged for detecting the spectrum of a reflected radiation beam coming from the sample of food product hit by the aforesaid radiation beam.

The spectrometer also comprises advantageously an operational unit, preferably provided with an electronic processor, arranged for receiving the measurements of the spectrum of the reflected beam detected by optical receiver and processing the detected data sets.

The apparatus according to the invention also comprises an electronic processing unit connected to the detection device and adapted to receive the first and the second set of detected data.

More in detail, the electronic processing unit comprises a first calculation module programmed for subjecting the first set of detected data to Principal Component Analysis (PCA). The first calculation module is thus advantageously programmed to obtain a plurality of principal components PCi, in which each principal component PCi has, associated therewith, a corresponding descriptive parameter Vi of the variability percentage of the first set of detected data.

The electronic processing unit also comprises a selection module programmed for selecting, from among the plurality of principal components Pci, at least one first principal component PC1 associated with the descriptive parameter Vi with maximum value.

In addition, the electronic processing unit comprises a second calculation module programmed for at least partially transforming the second set of detected data into a second modified data set by means of Principal Component Analysis (PCA) on the basis of the first principal component PC1. Therefore, the second calculation module is advantageously programmed in order to determine a second modified data set comprising a plurality of first principal component values PC1n, each associated with a corresponding electromagnetic spectrum of the second sequence.

The electronic processing unit also comprises a third calculation module programmed for calculating the standard deviation PC1dev of at least one set of first principal component values PC1n of the plurality of first principal component values PC1n and for calculating, on the basis of the standard deviation PC1dev, a confidence interval I.

In particular, the third calculation module is programmed for dividing the second sequence of electromagnetic spectra into sequences, obtaining different sets of first principal component values PC1n and advantageously calculating standard deviation PC1dev and confidence interval I for each of these. The electronic processing unit also comprises a comparison module programmed for comparing the set of first principal component values PC1n with the confidence interval I in order to determine the count number C of the first principal component values PC1n falling within the confidence interval I. Advantageously, the comparison module is programmed for executing the aforesaid comparison for each set of first principal component values PC1n.

The electronic processing unit also comprises a stopping module programmed for commanding the stop of the apparatus for mixing following at least the attainment, preferably the exceeding, of a pre-established threshold number S by the count number C.

Preferably, the stop module is programmed for commanding the stop of the apparatus upon attainment of a minimum limit of sets of first principal component values PC1n, preferably one after the other, having the count number C greater than or equal to the pre-established threshold number S.

Preferably, the electronic processing unit also comprises a safety control module programmed for counting the number of electromagnetic spectra of the second sequence and stopping the apparatus for mixing if the number of electromagnetic spectra exceeds preset maximum safety value.

Of course, the electronic processing unit can comprise further modules configured for executing other steps and stages of the above-described process.

Advantageously, the electronic processing unit comprises a circuit board, preferably provided with microprocessor.

Preferably, the processing unit of the apparatus and the operational unit of the detection device are made in a same circuit board or, otherwise, in separate circuit boards.

Advantageously, the apparatus, object of the present invention, is intended to be mounted on a mixer-wagon and preferably to detect the parameters relative to the different samples of food product in the mixer-wagon or along the front of the trough during the advancement of the mixer-wagon in the corridor of the stall, along which the trough itself is placed.

The apparatus, in this manner, is capable of evaluating, in an automated manner, the quality of the mixing and quickly and autonomously deciding whether to continue mixing the food product or stop the mixing. The invention thus described therefore attains the pre-established objects.

**Claims**

1. Process for mixing a food product for farm animals, said process being **characterized in that** it comprises:

   - a step of arranging a food product to be mixed within an apparatus for mixing;
   - a mixing step, in which said apparatus for mixing is actuated in order to mix said food product;
   - a first step of detecting a first sequence of electromagnetic spectra reflected by said food product, leading to the acquisition of a first set of detected data characteristic of the electromagnetic spectra of said first sequence;
   - a first calculation step, in which said first set of detected data is subjected to Principal Component Analysis (PCA) in order to obtain a plurality of principal components (PCi), in which each principal component (PCi) has, associated therewith, a corresponding descriptive parameter (Vi) of the variability percentage of said first set of detected data;
   - a selection step, in which between said plurality of principal components (PCi), at least one first principal component (PC1) is selected that is associated with the descriptive parameter (Vi) with maximum value;
   - a second step of detecting a second sequence of electromagnetic spectra reflected by said food product, leading to the acquisition of a second set of detected data characteristic of the electromagnetic spectra of said second sequence;
   - a second calculation step, in which said second set of detected data is at least partially transformed into a second modified data set by means of Principal Component Analysis (PCA) on the basis of said selected first principal component (PC1); said second modified data set comprising a plurality of first principal component values (PC1n), each associated with a corresponding electromagnetic spectrum of said second sequence;
   - a third calculation step, in which the standard deviation (PC1dev) is calculated of at least one set of first principal component values (PC1n) of said plurality of first principal component values (PC1n) and, on the basis of said standard deviation (PC1dev), a confidence interval (I) is calculated;
   - a step of comparing said set of first principal component values (PC1n) with said confidence interval (I) in order to determine the count number (C) of the first principal component values (PC1n) falling within said confidence interval (I);
   - a step of stopping the mixing of said apparatus for mixing following at least the attainment of a threshold number (S) pre-established by said count number (C).

2. Process according to claim 1, **characterized in that** said reflection electromagnetic spectra are electromagnetic spectra in the near infrared (NIR) wavelengths.

3. Process according to claim 1 or 2, **characterized in that** the electromagnetic spectra of said first and second sequence are detected in consecutive and equidistant time instants.

4. Process according to any one of the preceding claims, **characterized in that** said second detection step is executed after said first detection step.

5. Process according to any one of the preceding claims, **characterized in that** in said third calculation step:

- said second sequence of electromagnetic spectra is divided into a plurality of sub-sequences, each corresponding to a different said set of first principal component values (PC1n);
- said standard deviation (PC1dev) is calculated for the first principal component values (PC1n) of each said set of first principal component values (PC1n);
- said confidence interval (I) is established on the basis of each said standard deviation (PC1dev) associated with the corresponding said set of first principal component values (PC1n);

in which, in said comparison step, said count number (C) is determined for each corresponding said confidence interval (I) of each set of first principal component values (PC1n).

6. Process according to claim 5, **characterized in that** in said stopping step, the mixing is stopped upon attainment of a minimum limit of set of first principal component values (PC1n) having said count number (C) greater than or equal to the pre-established threshold number (S).

7. Process according to claim 5 or 6, **characterized in that** in said decision step, said pre-established threshold number (S) is equal to the number of electromagnetic spectra of said sub-sequence.

8. Process according to any one of the preceding claims, **characterized in that** at least said second detection step is executed continuously during the mixing of said food product;
in which said process comprises a step of safety control, at least partially executed simultaneously with said second detection step, in which the number of electromagnetic spectra of said second sequence is counted and in which said apparatus for mixing is stopped if said number of electromagnetic spectra exceeds a preset maximum safety value.

9. Process according to any one of the preceding claims, **characterized in that** the ends of said confidence interval (I) are obtained by summing and subtracting a margin of error (E) from the mean of the first principal component values (PC1n);

in which, in said third calculation step, the mean of the set of first principal component values (PC1n) is initially calculated and subsequently said margin of error (E) is calculated with the following formula:

$$E = Z_{\alpha/2} \cdot \frac{PC1dev}{\sqrt{(n)}}$$

in which $Z_{\alpha/2}$ is a confidence coefficient dependent on a confidence percentage ($\alpha$), PC1dev is the standard deviation and n is the number of first principal component values (PC1n) in the set of first principal component values (PC1n).

10. Process according to claim 9, **characterized in that** said confidence percentage ($\alpha$) is greater than 80%, preferably greater than 90%.

11. Process according to any one of the preceding claims, **characterized in that** it comprises a first and a second step of sampling the food product, in which multiple first and second samples corresponding to food product portions are identified;

in which the first and second samples of said food product are identified within said mixing apparatus during the mixing of said food product;

in which the electromagnetic spectra of said first and second sequence are detected on the corresponding first and second samples of said food product.

**12.** Apparatus for mixing a food product for farm animals, said apparatus comprising:

- a mixing tank in which a food product to be mixed is intended to be placed;
- mixing means placed within said mixing tank and actuatable in order to mix said food product;
- a detection device arranged for detecting at least one first and a second sequence of electromagnetic spectra reflected by said food product, and acquiring a first and a second set of detected data characteristic of the electromagnetic spectra respectively of said first and second sequence;
- an electronic processing unit connected to said detection device and adapted to receive said first and second set of detected data;

said apparatus being **characterized in that** said electronic processing unit comprises:

- a first calculation module programmed for subjecting said first set of detected data to Principal Component Analysis (PCA) and obtaining a plurality of principal components (PCi), in which each principal component (PCi) has, associated therewith, a corresponding descriptive parameter (Vi) of the variability percentage of said first set of detected data;
- a selection module programmed for selecting from among said plurality of principal components (PCi) at least one first principal component (PC1) associated with the descriptive parameter (Vi) with maximum value;
- a second calculation module programmed for at least partially transforming said second set of detected data into a second modified data set by means of Principal Component Analysis (PCA) on the basis of said first principal component (PC1); said second modified data set comprising a plurality of first principal component values (PC1n), each associated with a corresponding electromagnetic spectrum of said second sequence;
- a third calculation module programmed for calculating the standard deviation (PC1dev) of at least one set of first principal component values (PC1n) of said plurality of first principal component values (PC1n) and for calculating, on the basis of said standard deviation (PC1dev), a confidence interval (I);
- a comparison module programmed for comparing said set of first principal component values (PC1n) with said confidence interval (I) in order to determine the count number (C) of the first principal component values (PC1n) falling within said confidence interval (I);
- a programmed stopping module for commanding the stop of said apparatus for mixing following the attainment of a threshold number (S) pre-established by said count number (C).

**13.** Apparatus according to claim 12 **characterized in that** said detection device comprises an NIR spectrometer operating in the near infrared spectrum.

**14.** Apparatus according to claim 12 or 13, **characterized in that** said electronic processing unit comprises a safety control module programmed for counting the number of electromagnetic spectra of said second sequence and stopping said apparatus for mixing if said number of electromagnetic spectra exceeds a preset maximum safety value.

**15.** Apparatus according to any one of the claims from 12 to 14, **characterized in that** it is intended to be mounted on a mixer-wagon.

**Patentansprüche**

**1.** Verfahren zum Mischen eines Nahrungsmittelprodukts für Nutztiere, wobei das genannte Verfahren **dadurch gekennzeichnet ist, dass** es Folgendes umfasst:

- einen Schritt der Vorbereitung eines im Inneren einer Vorrichtung zum Mischen zu mischenden Nahrungsmittelprodukts;
- einen Mischschritt, bei dem die genannte Vorrichtung zum Mischen in Gang gesetzt wird, um das genannte Nahrungsmittelprodukt zu mischen;
- einen ersten Schritt des Messens einer ersten Folge von elektromagnetischen Spektren, die von dem genannten Nahrungsmittelprodukt reflektiert werden, der zur Erfassung einer ersten Menge von gemessenen Daten führt, die für die elektromagnetischen Spektren der genannten ersten Folge charakteristisch sind;
- einen ersten Berechnungsschritt, bei dem die genannte erste Menge gemessener Daten einer Hauptkomponentenanalyse (PCA) unterzogen wird, um eine Vielzahl von Hauptkomponenten (PCi) zu erhalten, bei dem jede Hauptkomponente (PCi) mit einem entsprechenden beschreibenden Parameter (Vi) des Prozentsatzes der Variabilität der genannten ersten Menge gemessener Daten verknüpft ist;

- einen Auswahlschritt, bei dem unter der genannten Vielzahl von Hauptkomponenten (PCi) mindestens eine mit dem beschreibenden Parameter (Vi) mit höchstem Wert verknüpfte erste Hauptkomponente (PC1) gewählt wird;

- einen zweiten Schritt des Messens einer zweiten Folge von elektromagnetischen Spektren, die von dem genannten Nahrungsmittelprodukt reflektiert werden, der zur Erfassung einer zweiten Menge gemessener Daten führt, die für die elektromagnetischen Spektren der genannten zweite Folge charakteristisch sind;

- einen zweiten Berechnungsschritt, bei dem die genannte zweite Menge gemessener Daten mindestens teilweise in eine zweite Datenmenge verwandelt wird, die mittels Hauptkomponentenanalyse (PCA) auf Grundlage der genannten gewählten ersten Hauptkomponente (PC1) verändert wird; wobei die genannte veränderte zweite Datenmenge eine Vielzahl von Werten (PC1n) der ersten Hauptkomponente umfasst, von denen jeder mit einem entsprechenden elektromagnetischen Spektrum der genannten zweiten Folge verknüpft ist;

- einen dritten Berechnungsschritt, bei dem die Standardabweichung (PC1dev) mindestens eines Satzes von Werten (PC1n) der ersten Hauptkomponente der genannten Vielzahl von Werten (PC1n) der ersten Hauptkomponente berechnet wird und auf Grundlage der genannten Standardabweichung (PC1dev) ein Vertrauensintervall (I) berechnet wird;

- einen Schritt des Vergleichs des genannten Satzes von Werten (PC1n) der ersten Hauptkomponente mit dem genannten Vertrauensintervall (I), um die Zählungsanzahl (C) der Werte (PC1n) der ersten Hauptkomponente zu bestimmen, die in das genannte Vertrauensintervall (I) fallen;

- einen Stoppschritt des Mischens der genannten Vorrichtung zum Mischen mindestens im Anschluss an das Erreichen einer zuvor durch die genannte Zählungsanzahl (C) festgelegten Schwellenanzahl (S).

2. Verfahren nach Anspruch Nr. 1, **dadurch gekennzeichnet, dass** die genannten elektromagnetischen Reflexionsspektren elektromagnetische Spektren in den Wellenlängen des Nahinfrarots (NIR) sind.

3. Verfahren nach Anspruch Nr. 1 oder 2, **dadurch gekennzeichnet, dass** die elektromagnetischen Spektren der genannten ersten und zweiten Folge zu aufeinanderfolgenden Zeitpunkten im gleichen Abstand gemessen werden.

4. Verfahren nach einem beliebigen der vorangegangenen Ansprüche, **dadurch gekennzeichnet, dass** der genannte zweite Messschritt im Anschluss an den genannten ersten Messschritt ausgeführt wird.

5. Verfahren nach einem beliebigen der vorangegangenen Ansprüche, **dadurch gekennzeichnet, dass** bei dem genannten dritten Berechnungsschritt:

- die genannte zweite Folge von elektromagnetischen Spektren in eine Vielzahl von Subfolgen unterteilt wird, die jeweils einem unterschiedlichen genannten Satz von Werten (PC1n) der ersten Hauptkomponente entsprechen;
- wobei die genannte Standardabweichung (PC1dev) für die Werte (PC1n) der ersten Hauptkomponente jedes genannten Satzes von Werten (PC1n) der ersten Hauptkomponente berechnet wird;
- wobei das genannte Vertrauensintervall (I) auf Grundlage der genannten Standardabweichung (PC1dev) festgelegt wird, die mit dem entsprechenden genannten Satz von Werten (PC1n) der ersten Hauptkomponente verknüpft ist;

wobei bei dem genannten Vergleichsschritt die genannte Zählungsanzahl (C) für jedes entsprechende genannte Vertrauensintervall (I) jedes Satzes von Werten (PC1n) der ersten Hauptkomponente bestimmt wird.

6. Verfahren nach Anspruch Nr. 5, **dadurch gekennzeichnet, dass** bei dem genannten Stoppschritt das Mischen beim Erreichen eines Mindestgrenzwerts des Satzes von Werten (PC1n) der ersten Hauptkomponente gestoppt wird, deren Zählungsanzahl (C) mindestens der zuvor festgelegten Schwellenanzahl (S) entspricht.

7. Verfahren nach Anspruch Nr. 5 oder 6, **dadurch gekennzeichnet, dass** bei dem genannten Entscheidungsschritt die genannte zuvor festgelegte Schwellenanzahl (S) der Anzahl der elektromagnetischen Spektren der genannten Subfolge entspricht.

8. Verfahren nach einem beliebigen der vorangegangenen Ansprüche, **dadurch gekennzeichnet, dass** mindestens der genannte zweite Messschritt während des Mischens des genannten Nahrungsmittelprodukts kontinuierlich ausgeführt wird;

wobei das genannte Verfahren einen Sicherheitskontrollschritt umfasst, der mindestens teilweise zeitgleich mit dem genannten zweiten Messschritt ausgeführt wird, bei dem die Anzahl der elektromagnetischen Spektren der genannten zweiten Folge gezählt wird und bei dem die genannte Vorrichtung zum Mischen gestoppt wird, wenn die genannte Anzahl elektromagnetischer Spektren einen voreingestellten maximalen Sicherheitswert überschreitet.

9. Verfahren nach einem beliebigen der vorangegangenen Ansprüche, **dadurch gekennzeichnet, dass** die Daten des genannten Vertrauensintervalls (I) durch Addieren und Subtrahieren einer Fehlermarge (E) zum oder vom Durchschnitt der Werte (PC1n) der ersten Hauptkomponente erhalten werden;

wobei bei dem genannten dritten Berechnungsschritt anfänglich der Durchschnitt des Satzes von Werten (PC1n) der ersten Hauptkomponente berechnet und anschließend die genannte Fehlermarge (E) mit der folgenden Formel berechnet wird:

$$E = Z_{\alpha/2} \cdot \frac{PC1dev}{\sqrt{(n)}}$$

wobei $Z_{\alpha/2}$ ein Vertrauenskoeffizient ist, der von einem Vertrauensprozentsatz ($\alpha$) abhängt, PC1dev die Standardabweichung und n die Anzahl der Werte (PC1n) der ersten Hauptkomponente im Satz der Werte (PC1n) der ersten Hauptkomponente ist.

10. Verfahren nach Anspruch Nr. 9, **dadurch gekennzeichnet, dass** der genannte Vertrauensprozentsatz ($\alpha$) mehr als 80 %, vorzugsweise mehr als 90 % beträgt.

11. Verfahren nach einem beliebigen der vorangegangenen Ansprüche, **dadurch gekennzeichnet, dass** das Verfahren einen ersten und einen zweiten Schritt der Stichprobennahme des Nahrungsmittelprodukts umfasst, bei denen mehrere erste und zweite Stichproben identifiziert werden, die Portionen des Nahrungsmittelprodukts entsprechen;

wobei die ersten und zweiten Stichproben des genannten Nahrungsmittelprodukts im Inneren der genannten Vorrichtung zum Mischen während des Mischens des genannten Nahrungsmittelprodukts identifiziert werden; wobei die elektromagnetischen Spektren der genannten ersten und zweiten Folge anhand von entsprechenden ersten und zweiten Stichproben des genannten Nahrungsmittelprodukts gemessen werden.

12. Vorrichtung zum Mischen eines Nahrungsmittelprodukts für Nutztiere, wobei die genannte Vorrichtung Folgendes umfasst:

- eine Mischwanne, in der ein zu mischendes Nahrungsmittelprodukt bestimmt ist, vorbereitet zu werden;
- im Inneren der genannten Mischwanne angeordnete Mischelemente, die in Gang gesetzt werden können, um das genannte Nahrungsmittelprodukt zu mischen;
- eine Messvorrichtung, die darauf ausgelegt ist, mindestens eine erste und eine zweite Folge von elektromagnetischen Spektren zu messen, die von dem genannten Nahrungsmittelprodukt reflektiert werden, und eine erste und eine zweite Menge gemessener Daten zu erfassen, die für die elektromagnetischen Spektren jeweils der genannten ersten und zweiten Folge charakteristisch sind;
- ein elektronisches Verarbeitungsgerät, das an die genannte Messvorrichtung angeschlossen und geeignet ist, die genannte erste und zweite Menge gemessener Daten zu empfangen;

wobei die genannte Vorrichtung **dadurch gekennzeichnet ist, dass** das genannte elektronische Verarbeitungsgerät Folgendes umfasst:

- ein erstes Berechnungsmodul, das dazu programmiert ist die genannte erste Menge gemessener Daten einer Hauptkomponentenanalyse (PCA) zu unterziehen und eine Vielzahl von Hauptkomponenten (PCi) zu erhalten, wobei jede Hauptkomponente (PCi) mit einem entsprechenden beschreibenden Parameter (Vi) des Prozentsatzes der Variabilität der genannten ersten Menge gemessener Daten verknüpft ist;
- ein Auswahlmodul, das dazu programmiert ist, unter der genannten Vielzahl von Hauptkomponenten (PCi) mindestens eine mit dem beschreibenden Parameter (Vi) mit höchstem Wert verknüpfte erste Hauptkomponente (PC1) zu wählen;
- ein zweites Berechnungsmodul, das dazu programmiert ist, die genannte zweite Menge gemessener Daten mindestens teilweise in eine zweite Datenmenge zu verwandeln, die mittels Hauptkomponentenanalyse (PCA) auf Grundlage der genannten ersten Hauptkomponente (PC1) geändert wird; wobei die genannte geänderte zweite Datenmenge eine Vielzahl von Werten (PC1n) der ersten Hauptkomponente umfasst, von denen jeder mit einem entsprechenden elektromagnetischen Spektrum der genannten zweiten Folge verknüpft ist;

- ein drittes Berechnungsmodul, das dazu programmiert ist, die Standardabweichung (PC1dev) mindestens eines Satzes von Werten (PC1n) der ersten Hauptkomponente der genannten Vielzahl von Werten (PC1n) der ersten Hauptkomponente zu berechnen und auf Grundlage der genannten Standardabweichung (PC1dev) ein Vertrauensintervall (I) zu berechnen;

- ein Vergleichsmodul, das dazu programmiert ist, den genannten Satz von Werten der ersten Hauptkomponente (PC1n) mit dem genannten Vertrauensintervall (I) zu vergleichen, um die Zählungsanzahl (C) der Werte (PC1n) der ersten Hauptkomponente zu bestimmen, die in das genannte Vertrauensintervall (I) fallen;

- ein Stoppmodul, das dazu programmiert ist, das Stoppen der genannten Vorrichtung zum Mischen im Anschluss an das Erreichen einer zuvor durch die genannte Zählungsanzahl (C) festgelegten Schwellenanzahl (S) zu steuern.

13. Vorrichtung nach Anspruch Nr. 12, **dadurch gekennzeichnet, dass** die genannte Messvorrichtung ein NIR-Spektrometer umfasst, das im Spektrum des Nahinfrarots arbeitet.

14. Vorrichtung nach Anspruch Nr. 12 oder 13, **dadurch gekennzeichnet, dass** das genannte elektronische Verarbeitungsgerät ein Sicherheitskontrollmodul umfasst, das dazu programmiert ist, die Anzahl der elektromagnetischen Spektren der genannten zweiten Folge zu zählen und die genannte Vorrichtung zum Mischen zu stoppen, wenn die genannte Anzahl elektromagnetischer Spektren einen voreingestellten maximalen Sicherheitswert überschreitet.

15. Vorrichtung nach einem beliebigen der Ansprüche 12 bis 14, **dadurch gekennzeichnet, dass** sie dazu bestimmt ist, auf einem Mischwagen montiert zu werden.


**Revendications**

1. Procédé permettant de mélanger un produit alimentaire pour des animaux d'élevage, ledit procédé étant **caractérisé en ce qu'**il comprend :

- une étape de placement d'un produit alimentaire à mélanger à l'intérieur d'un appareil de mélange ;
- une étape de mélange, dans laquelle ledit appareil de mélange est actionné pour mélanger ledit produit alimentaire ;
- une première étape de détection d'une première séquence de spectres électromagnétiques réfléchis par ledit produit alimentaire, conduisant à l'acquisition d'un premier groupe de données détectées caractéristiques des spectres électromagnétiques de ladite première séquence ;
- une première étape de calcul, dans laquelle ledit premier groupe de données détectées est soumis à l'analyse en composantes principales (PCA) pour obtenir une pluralité de composantes principales (PCi), dans lequel chaque composante principale (PCi) a associé un paramètre de description (Vi) correspondant du pourcentage de variabilité dudit premier groupe de données détectées ;
- une étape de sélection, dans laquelle au moins une première composante principale (PC1) associée au paramètre de description (Vi) avec la valeur maximale est sélectionnée parmi ladite pluralité de composantes principales (PCi) ;
- une deuxième étape de détection d'une deuxième séquence de spectres électromagnétiques réfléchis par ledit produit alimentaire, conduisant à l'acquisition d'un deuxième groupe de données détectées caractéristiques des spectres électromagnétiques de ladite deuxième séquence ;
- une deuxième étape de calcul, dans laquelle ledit deuxième groupe de données détectées est au moins partiellement transformé en un deuxième groupe de données modifiées par l'analyse en composantes principales (PCA) sur la base de ladite première composante principale (PC1) sélectionnée ; ledit deuxième groupe de données modifiées comprenant une pluralité de valeurs de première composante principale (PC1n) chacune associée à un spectre électromagnétique correspondant de ladite deuxième séquence ;
- une troisième étape de calcul, dans laquelle l'écart-type (PC1dev) d'au moins un ensemble de valeurs de première composante principale (PC1n) de ladite pluralité de valeurs de première composante principale (PC1n) est calculé et, sur la base dudit écart-type (PC1dev), un intervalle de confiance (I) est calculé ;
- une étape de comparaison dudit ensemble de valeurs de première composante principale (PC1n) avec ledit intervalle de confiance (I) pour déterminer le nombre de comptage (C) des valeurs de première composante principale (PC1n) tombant dans ledit intervalle de confiance (I) ;
- une étape d'arrêt du mélange dudit appareil de mélange au moins après avoir atteint un nombre seuil (S) prédéfini par ledit nombre de comptage (C).

2. Procédé selon la revendication 1, **caractérisé en ce que** lesdits spectres électromagnétiques de réflexion sont des spectres électromagnétiques appartenant au domaine des longueurs d'onde du proche infrarouge (NIR).

3. Procédé selon la revendication 1 ou 2, **caractérisé en ce que** les spectres électromagnétiques desdites première et deuxième séquences sont détectés à des instants temporels consécutifs et équidistants.

4. Procédé selon l'une quelconque des revendications précédentes, **caractérisé en ce que** ladite deuxième étape de détection est effectuée après ladite première étape de détection.

5. Procédé selon l'une quelconque des revendications précédentes, **caractérisé en ce qu'**à ladite troisième étape de calcul :

- ladite deuxième séquence de spectres électromagnétiques est divisée en une pluralité de sousséquences chacune correspondant à un ensemble différent de valeurs de première composante principale (PC1n) ;
- ledit écart-type (PC1dev) est calculé pour les valeurs de première composante principale (PC1n) de chaque ensemble de valeurs de première composante principale (PC1n) ;
- ledit intervalle de confiance (I) est établi sur la base de chaque écart-type (PC1dev) associé audit ensemble correspondant de valeurs de première composante principale (PC1n) ;

dans lequel, à ladite étape de comparaison, ledit nombre de comptage (C) est déterminé pour chaque intervalle de confiance (I) correspondant de chaque ensemble de valeurs de première composante principale (PC1n).

6. Procédé selon la revendication 5, **caractérisé en ce que**, à ladite étape d'arrêt, le mélange est arrêté après avoir atteint une limite minimale d'ensemble de valeurs de première composante principale (PC1n) ayant ledit nombre de comptage (C) supérieur ou égal au nombre seuil (S) prédéfini.

7. Procédé selon la revendication 5 ou 6, **caractérisé en ce que**, à ladite étape de décision, ledit nombre seuil (S) prédéfini est égal au nombre de spectres électromagnétiques de ladite sous-séquence.

8. Procédé selon l'une quelconque des revendications précédentes, **caractérisé en ce qu'**au moins ladite deuxième étape de détection est effectuée en continu pendant le mélange dudit produit alimentaire ; dans lequel ledit procédé comprend une étape de contrôle de sécurité, effectuée au moins en partie simultanément à ladite deuxième étape de détection, dans laquelle le nombre de spectres électromagnétiques de ladite deuxième séquence est compté et dans laquelle ledit appareil de mélange est arrêté si ledit nombre de spectres électromagnétiques dépasse une valeur maximale de sécurité préétablie.

9. Procédé selon l'une quelconque des revendications précédentes, **caractérisé en ce que** les valeurs extrêmes dudit intervalle de confiance (I) sont obtenues en additionnant et en soustrayant une marge d'erreur (E) à la moyenne des valeurs de première composante principale (PC1n) ;

dans lequel, à ladite troisième étape de calcul, la moyenne de l'ensemble de valeurs de première composante principale (PC1n) est tout d'abord calculée et, ensuite, ladite marge d'erreur (E) est calculée avec la formule suivante :

$$E = Z_{\alpha/2} \cdot \frac{PC1dev}{\sqrt{(n)}}$$

dans lequel $Z_{\alpha/2}$ est un coefficient de confiance qui dépend d'un pourcentage de confiance ($\alpha$), PC1dev est l'écart-type et n est le nombre de valeurs de première composante principale (PC1n) dans l'ensemble de valeurs de première composante principale (PC1n).

10. Procédé selon la revendication 9, **caractérisé en ce que** ledit pourcentage de confiance ($\alpha$) est supérieur à 80%, de préférence supérieur à 90%.

**11.** Procédé selon l'une quelconque des revendications précédentes, **caractérisé en ce qu'**il comprend une première et une deuxième étape d'échantillonnage du produit alimentaire, dans lesquelles plusieurs premiers et deuxièmes échantillons correspondant à des parties de produit alimentaire sont identifiés ;

dans lequel les premiers et deuxièmes échantillons dudit produit alimentaire sont identifiés à l'intérieur dudit appareil de mélange pendant le mélange dudit produit alimentaire ;

dans lequel les spectres électromagnétiques desdites première et deuxième séquences sont détectés sur les premiers et deuxièmes échantillons correspondants dudit produit alimentaire.

**12.** Appareil permettant de mélanger un produit alimentaire pour des animaux d'élevage, ledit appareil comprenant :

- une cuve de mélange dans laquelle un produit alimentaire à mélanger est destiné à être placé ;
- des moyens de mélange disposés à l'intérieur de ladite cuve de mélange et pouvant être actionnés pour mélanger ledit produit alimentaire ;
- un dispositif de détection prédisposé pour détecter au moins une première et une deuxième séquence de spectres électromagnétiques réfléchis par ledit produit alimentaire, et acquérir un premier et un deuxième groupe de données détectées caractéristiques des spectres électromagnétiques desdites première et deuxième séquences, respectivement ;
- une unité de traitement électronique reliée audit dispositif de détection et apte à recevoir lesdits premier et deuxième groupes de données détectées ;

ledit appareil étant **caractérisé en ce que** ladite unité de traitement électronique comprend :

- un premier module de calcul programmé pour soumettre ledit premier groupe de données détectées à l'analyse en composantes principales (PCA) et obtenir une pluralité de composantes principales (PCi), dans laquelle chaque composante principale (PCi) a associé un paramètre de description (Vi) correspondant du pourcentage de variabilité dudit premier groupe de données détectées ;
- un module de sélection programmé pour sélectionner au moins une première composante principale (PC1) associée au paramètre de description (Vi) avec la valeur maximale parmi ladite pluralité de composantes principales (PCi) ;
- un deuxième module de calcul programmé pour transformer au moins partiellement ledit deuxième groupe de données détectées en un deuxième groupe de données modifiées par l'analyse en composantes principales (PCA) sur la base de ladite première composante principale (PC1) ; ledit deuxième groupe de données modifiées comprenant une pluralité de valeurs de première composante principale (PC1n) chacune associée à un spectre électromagnétique correspondant de ladite deuxième séquence ;
- un troisième module de calcul programmé pour calculer l'écart-type (PC1dev) d'au moins un ensemble de valeurs de première composante principale (PC1n) de ladite pluralité de valeurs de première composante principale (PC1n) et pour calculer, sur la base dudit écart-type (PC1dev), un intervalle de confiance (I) ;
- un module de comparaison programmé pour comparer ledit ensemble de valeurs de première composante principale (PC1n) avec ledit intervalle de confiance (I) pour déterminer le nombre de comptage (C) des valeurs de première composante principale (PC1n) tombant dans ledit intervalle de confiance (I) ;
- un module d'arrêt programmé pour commander l'arrêt dudit appareil de mélange après avoir atteint un nombre seuil (S) prédéfini par ledit nombre de comptage (C).

**13.** Appareil selon la revendication 12, **caractérisé en ce que** ledit dispositif de détection comprend un spectromètre NIR agissant dans le spectre du proche infrarouge.

**14.** Appareil selon la revendication 12 ou 13, **caractérisé en ce que** ladite unité de traitement électronique comprend un module de contrôle de sécurité programmé pour compter le nombre de spectres électromagnétiques de ladite deuxième séquence et arrêter ledit appareil de mélange si ledit nombre de spectres électromagnétiques dépasse une valeur maximale de sécurité préétablie.

**15.** Appareil selon l'une quelconque des revendications 12 à 14, **caractérisé en ce qu'**il est destiné à être monté sur un wagon mélangeur.

Fig. 1

Fig. 2b

Fig. 2d

Fig. 2a

Fig. 2c

Fig. 2f

Fig. 2e

Fig. 2h

Fig. 2g

EP 4 223 147 B1

Farm3, day 6

**Fig. 2i**

Farm 3, day 7

**Fig. 2j**

Farm 3, day 8

**Fig. 2k**

Farm 3, day 10

**Fig. 2m**

Farm 3, day 11

**Fig. 2n**

Farm 3, day 9

**Fig. 2l**

Farm 1, day 1

**Fig. 2o**

Farm 2, day 1

**Fig. 2p**

Farm 3, day 1

**Fig. 2q**

Farm 3, day 2

**Fig. 2r**

Farm 3, day 3

log(1/R)

wavelength (nm)

**Fig. 2s**

Farm 3, day 4

log(1/R)

wavelength (nm)

**Fig. 2t**

Farm 3, day 5

log(1/R)

wavelength (nm)

**Fig. 2u**

Farm 3, day 6

log(1/R)

wavelength (nm)

**Fig. 2v**

Farm 3, day 7

log(1/R)

wavelength (nm)

**Fig. 2w**

Farm 1, day 1

Fig. 3a

Farm 2, day 1

Fig. 3b

Farm 3, day 1

Fig. 3c

Farm 3, day 2

Fig. 3d

Fig. 3e

Fig. 3f

Fig. 3g

Fig. 3h

Fig. 3i

Farm 1, day 1

Farm 2, day 1

**Fig. 4a**

**Fig. 4b**

Farm 3, day1

Farm 3, day 2

**Fig. 4c**

**Fig. 4d**

Farm 3, day 3

Farm 3, day 4

**Fig. 4e**

**Fig. 4f**

Farm 3, day 5

Farm 3, day 6

Farm 3, day 7

**Fig. 4g**

**Fig. 4h**

**Fig. 4i**

Fig. 5a

Fig. 5b

Fig. 5c

Fig. 5d

Fig. 5e

Fig. 5f

Fig. 5g

Fig. 5h

Fig. 5i

**Fig. 6a** — Farm 1, day 1

**Fig. 6b** — Farm 2, day 1

**Fig. 6c** — Farm 3, day 1

**Fig. 6d** — Farm 3, day 2

Fig. 6e

Fig. 6f

Fig. 6g

Fig. 6h

Fig. 6i

Fig. 7a

Fig. 7b

Fig. 7c

Fig. 7d

Farm 3, day 4

**Fig. 7f**

Farm 3, day 7

**Fig. 7i**

Farm 3, day 3

**Fig. 7e**

Farm 3, day 6

**Fig. 7h**

Farm 3, day 5

**Fig. 7g**

Fig. 8a

Fig. 8b

Fig. 8c

Fig. 8d

Farm 3, day 4

Fig. 8f

Farm 3, day 3

Fig. 8e

Farm 3, day 7

Fig. 8i

Farm 3, day 6

Fig. 8h

Farm 3, day 5

Fig. 8g

Fig. 9b

Fig. 9d

Fig. 9a

Fig. 9c

Fig. 9e

Fig. 9f

Fig. 9g

Fig. 9h

Fig. 9i

**Fig. 10b**

**Fig. 10d**

**Fig. 10a**

**Fig. 10c**

Fig. 10f

Fig. 10e

Fig. 10i

Fig. 10h

Fig. 10g

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- DE 102010033886

**Non-patent literature cited in the description**

- **LAMMERS B.P ; BUCKMASTER D.R ; HEINRICHS A.J.** A simple method for the analysis of particle size of forage and total mixed rations. *Journal of Dairy Science,* 1996, vol. 79, 922-928